(19) Europäisches Patentamt
European Patent Office
Office européen des brevets

(11) Veröffentlichungsnummer: **0 009 008**
**A2**

(12) # EUROPÄISCHE PATENTANMELDUNG

(21) Anmeldenummer: 79810085.5

(22) Anmeldetag: 03.09.79

(51) Int. Cl.³: C 07 D 501/00
C 07 D 501/59, A 61 K 31/545
//C07D277/38

(30) Priorität: 08.09.78 CH 9468/78

(43) Veröffentlichungstag der Anmeldung: 19.03.80 Patentblatt 80/6

(84) Benannte Vertragsstaaten: AT BE CH DE FR GB IT LU NL SE

(71) Anmelder: CIBA-GEIGY AG
Patentabteilung Postfach
CH-4002 Basel(CH)

(72) Erfinder: Schneider, Peter, Dr.
Rheinfelderstrasse 21 A/1
CH-4058 Basel(CH)

(72) Erfinder: Wiederkehr, René, Dr.
Grenzweg 9
CH-4148 Pfeffingen(CH)

(72) Erfinder: Wenk, Paul, Dr.
Karl Jaspers Allee 40
CH-4052 Basel(CH)

(72) Erfinder: Scartazzini, Riccardo, Dr.
Conrad-Ferdinand-Meyer-Strasse 38
CH-4059 Basel(CH)

(54) **Cephalosporinderivate, Verfahren zu ihrer Herstellung und diese enthaltende pharmazeutische Präparate.**

(57) 7β-Aminothiazolyl-acetamido-3-cephem-4-carbonsäure-verbindungen der Formel

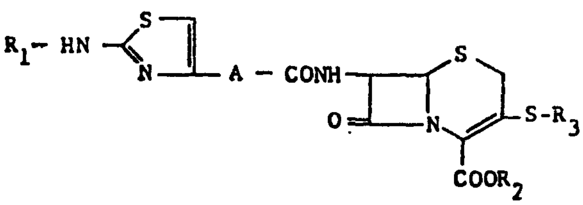

(I)

worin $R_1$ Wasserstoff, Niederalkyl oder eine Aminoschutzgruppe, A Methylen oder durch gegebenenfalls geschütztes Amino, Hydroxy, gegebenenfalls geschütztes Sulfo, Oxo, gegebenenfalls geschütztes Hydroxyimino oder Methoxyimino substituiertes Methylen, $R_2$ Wasserstoff oder einen die Carboxylgruppe veresternden Rest und $R_3$ einen Heterocyclylrest darstellen, und Salze von solchen Verbindungen mit salzbildenden Gruppen, Verfahren zu ihrer Herstellung und pharmazeutische Präparate enthaltend die aktiven Verbindungen.

Die Verbindungen haben antibiotische Wirksamkeit und können zur Bekämpfung von Infektionen verwendet werden.

EP 0 009 008 A2

Croydon Printing Company Ltd

CIBA-GEIGY AG,
Basel (Schweiz)

4-12024/+

# 3-Substituierte Aminothiazolverbindungen

Die Erfindung betrifft neue 7β-Aminothiazolyl-acetamido-3-cephem-4-carbonsäure-verbindungen, die in 3-Stellung des Cephemringes durch eine Heterocyclylthiogruppe substituiert sind, Verfahren zu ihrer Herstellung, pharmazeutische Präparate die solche Verbindungen enthalten und ihre Verwendung als Antibiotika.

Die Erfindung betrifft insbesondere 7β-Aminothiazolyl-acetamido-3-cephem-4-carbonsäure-verbindungen der Formel

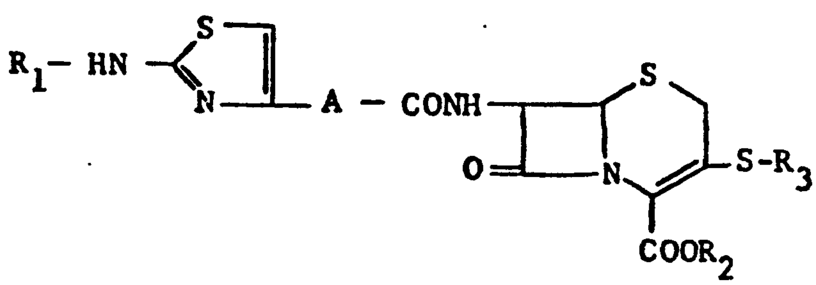

(I)

worin $R_1$ Wasserstoff, Niederalkyl oder eine Aminoschutzgruppe, A Methylen oder durch gegebenenfalls geschütztes Amino, Hydroxy, gegebenenfalls geschütztes Sulfo, Oxo, gegebenenfalls geschütztes Hydroxyimino oder Methoxyimino substituiertes Methylen, $R_2$ Wasserstoff oder einen die Carboxylgruppe veresternden Rest und $R_3$ einen Heterocyclylrest darstellen, und Salze von solchen Verbindungen mit salzbildenden Gruppen.

Die erfindungsgemässen Verbindungen der Formel I können auch in ihrer tautomeren Form, d.h. als Imino-

thiazolinyl-verbindungen der Formel

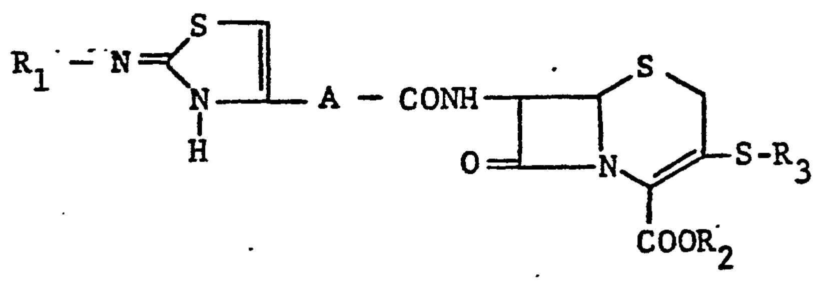

(IA)

oder auch als Tautomerengemische vorliegen. Die Tautomeren der Formel IA und die Tautomerengemische fallen ebenfalls unter den Umfang der vorliegenden Erfindung. Der Einfachheit wegen werden die Verbindungen hiernach aber nur in der Aminothiazolylform geschrieben.

In der vorliegenden Beschreibung der Erfindung bedeutet der Ausdruck "Nieder" in Gruppen wie Niederalkyl, Niederalkylen, Niederalkoxy, Niederalkanoyl und dergleichen, dass die entsprechenden Gruppen, sofern nicht ausdrücklich anders definiert, bis zu 7, bevorzugt bis zu 4 C-Atome enthalten.

Eine Niederalkylgruppe $R_1$ ist insbesondere $C_1$-$C_4$-Niederalkyl, wie Methyl, Aethyl, Propyl, Isopropyl, Butyl oder tert.-Butyl.

Die in Verbindungen der Formel I vorhandenen funktionellen Gruppen, insbesondere Carboxyl- und Amino-, Hydroxy-, Hydroxyimino- und Sulfogruppen, sind gegebenenfalls durch Schutzgruppen geschützt, die in der Penicillin-, Cephalosporin- und Peptidchemie verwendet werden.

Solche Schutzgruppen sind leicht, das heisst ohne dass unerwünschte Nebenreaktionen stattfinden, beispielsweise solvolytisch, reduktiv, photolytisch oder auch unter physiologischen Bedingungen, abspaltbar.

Schutzgruppen dieser Art, sowie ihre Abspaltung sind beispielsweise beschrieben in "Protective Groups in Organic Chemistry", Plenum Press, London, New York,

1973, ferner in "The Peptides", Vol. I, Schröder and Lübke, Academic Press, London, New York, 1965, sowie in Houben-Weyl, Methoden der organischen Chemie, 4. Aufl., Bd. 15/1, Georg Thieme Verlag, Stuttgart, 1974.

Eine Aminoschutzgruppe $R_1$ ist in erster Linie eine Acylgruppe Ac, eine Mono-, Di- oder Triarylmethylgruppe oder eine Silyl- oder Stannylgruppe.

Eine Acylgruppe Ac ist beispielsweise der Acylrest einer organischen Carbonsäure mit z.B. bis zu 18 C-Atomen, insbesondere einer gegebenenfalls, z.B. durch Halogen oder Aryl, substituierten aliphatischen Carbonsäure oder gegebenenfalls, z.B. durch Halogen, Niederalkoxy oder Nitro, substituierten aromatischen Carbonsäure, oder eines Kohlensäurehalbesters. Solche Acylgruppen sind beispielsweise Niederalkanoyl, wie Formyl, Acetyl, Propionyl, Halogenniederalkanoyl, wie 2-Halogenacetyl, insbesondere 2-Chlor-, 2-Brom-, 2-Jod-, 2,2-Dichlor- oder 2,2,2-Trichloracetyl, Phenylacetyl, Phenoxyacetyl, Thienylacetyl, Benzoyl, 4-Chlor-, 4-Methoxy- oder 4-Nitrobenzoyl, Niederalkoxycarbonyl, insbesondere tert.-Niederalkoxycarbonyl, z.B. tert.-Butyloxycarbonyl, Polycycloalkoxycarbonyl, z.B. Adamantyloxycarbonyl, Arylmethoxycarbonyl, worin Aryl vorzugsweise einen oder zwei, gegebenenfalls, z.B. durch Niederalkyl, insbesondere tert.-Niederalkyl, z.B. tert.-Butyl, Niederalkoxy, wie Methoxy, Hydroxy, Halogen, z.B. Chlor, und/oder Nitro, mono- oder polysubstituierte Phenylreste darstellt, wie gegebenenfalls substituiertes Benzyloxycarbonyl, z.B. 4-Nitro-benzyloxycarbonyl, oder substituiertes Diphenylmethoxycarbonyl, z.B. Benzhydryloxycarbonyl oder Di-(4-methoxyphenyl)-methoxycarbonyl, oder 2-Halogen-niederalkoxycarbonyl, z.B. 2,2,2-Trichloräthoxycarbonyl, 2-Chloräthoxycarbonyl, 2-Brom-

äthoxycarbonyl oder 2-Jodäthoxycarbonyl, 2-($S_1$)($S_2$)($S_3$)-Silyläthoxycarbonyl, worin die Substituenten $S_1$, $S_2$ und $S_3$ unabhängig voneinander je einen gegebenenfalls substituierten, z.B. durch Niederalkyl, Niederalkoxy, Aryl, Halogen oder Nitro, substituierten, aliphatischen, araliphatischen, cycloaliphatischen oder aromatischen Kohlenwasserstoffrest mit z.B. bis zu 15 C-Atomen, wie einen entsprechenden gegebenenfalls substituierten Niederalkyl-, Arylniederalkyl-, Cycloalkyl- oder Arylrest, bedeuten, z.B. 2-Triniederalkylsilyläthoxycarbonyl, wie 2-Trimethylsilyläthoxycarbonyl oder 2-(Dibutyl-methyl-silyl)-äthoxycarbonyl, oder 2-Triarylsilyläthoxycarbonyl, wie 2-Triphenylsilyläthoxycarbonyl, oder Acylmethoxycarbonyl, wie Aroylmethoxycarbonyl, worin die Aroylgruppe vorzugsweise gegebenenfalls, z.B. durch Halogen, wie Brom, substituiertes Benzoyl darstellt, z.B. Phenacyloxycarbonyl.

Weitere Acylgruppen Ac sind Reste organischer Phosphor-, Phosphon- oder Phosphinsäuren der Formel $(R^1)(R^2)P(=O)-$, worin $R^1$ und $R^2$ unabhängig voneinander eine durch einen Kohlenwasserstoffrest verätherte Hydroxygruppe oder einen Kohlenwasserstoffrest bedeuten, wobei die Kohlenwasserstoffreste bevorzugt bis zu 15 C-Atome enthalten, beispielsweise aliphatischer, araliphatischer, cycloaliphatischer oder aromatischer Natur sind, gegebenenfalls z.B. durch Niederalkyl, Niederalkoxy, Aryl, Halogen oder Nitro substituiert sind, und beispielsweise Alkyl, insbesondere Niederalkyl, wie Methyl, Aethyl, Propyl, Isopropyl oder Butyl, Phenylniederalkyl, wie Benzyl, p-Nitro- oder p-Chlorbenzyl, Cycloalkyl, wie Cyclopropyl, Cyclopentyl, Cyclohexyl oder Cycloheptyl, oder Homoaryl, wie Phenyl, o-, m- oder p-Tolyl, p-Methoxyphenyl, p-Biphenylyl, p-Chlorphenyl oder p-Nitrophenyl, bedeuten. Solche Reste sind beispielsweise Dimethylphosphoryl, Diäthylphosphoryl, Dipropylphosphoryl, Diisopropylphosphoryl, Dibenzylphosphoryl, Di-p-nitrobenzylphosphoryl, Dicyclohexylphosphoryl, Diphenylphosphoryl, Phenoxy-phenylphosphonyl, Diäthylphosphinyl und Diphenylphosphinyl.

In einer Mono-, Di- oder Triarylmethylgruppe $R_1$ sind die Arylreste insbesondere gegebenenfalls substituierte Phenylreste. Solche Gruppen sind beispielsweise Benzyl, Diphenylmethyl oder Trityl.

Eine Silyl- oder Stannylgruppe $R_1$ ist in erster Linie eine organische Silyl- bzw. Stannylgruppe, worin das Silicium- bzw. Zinnatom vorzugsweise Niederalkyl, insbesondere Methyl, ferner Niederalkoxy, z.B. Methoxy, und/oder Halogen, z.B. Chlor, als Substituenten enthält. Entsprechende Silyl- oder Stannylgruppen sind in erster Linie Triniederalkylsilyl, insbesondere Trimethylsilyl, ferner Dimethyl-tert.-butyl-silyl, Niederalkoxyniederalkyl-halogen-silyl, z.B. Methoxy-methyl-chlor-silyl, oder Diniederalkyl-halogen-silyl, z.B. Dimethyl-chlorsilyl, oder entsprechend substituiertes Stannyl, z.B. Tri-n-butylstannyl.

Bevorzugte Aminoschutzgruppen $R_1$ sind die Acylreste der 2-Halogenessigsäure, insbesondere 2-Chloracetyl, sowie von Kohlensäurehalbestern, insbesondere tert.-Niederalkoxycarbonyl, z.B. tert. Butyloxycarbonyl, gegebenenfalls, z.B. wie angegeben substituiertes Benzyloxycarbonyl oder Diphenylmethoxycarbonyl, 2-Halogen-niederalkoxycarbonyl, wie 2,2,2-Trichloräthoxycarbonyl, 2-Triniederalkylsilyläthoxycarbonyl, wie 2-Trimethylsilyläthoxycarbonyl, sowie die Tritylgruppe.

Die gleichen Aminoschutzgruppen können in einer im Rest $R_3$ oder A befindliche geschützten Aminogruppe

vorhanden sein.

Eine Aminogruppe kann auch in protonierter Form geschützt werden; als Anionen kommen in erster Linie diejenigen von starken anorganischen Säuren, wie von Halogenwasserstoffsäuren, z.B. das Chlor- oder Bromanion, oder von Sulfonsäuren, wie p-Toluolsulfonsäure, in Frage.

Die in geschützten Hydroxy- oder Hydroxyimino-gruppen im Rest $R_3$ oder A vorhandenen Hydroxyschutzgruppen sind z.B. die im Zusammenhang mit einer geschützten Aminogruppe genannten Acylreste Ac, wie 2-Halogenacetyl, z.B. 2-Chlor- oder 2,2-Dichloracetyl, oder insbesondere Acylreste von Kohlensäurehalbestern, insbesondere 2,2,2-Trichloräthoxycarbonyl, oder organische Silyl- oder Stannylreste, ferner leicht abspaltbare 2-oxa- oder 2-thia-aliphatische oder -cycloaliphatische Kohlenwasserstoffreste, in erster Linie 1-Niederalkoxy-niederalkyl oder 1-Niederalkylthio-niederalkyl, z.B. 1-Methoxy-äthyl, 1-Aethoxy-äthyl, 1-Methylthio-äthyl oder 1-Aethylthio-äthyl, oder 2-Oxa- oder 2-Thiacycloniederalkyl mit 5-7 Ringatomen, z.B. 2-Tetrahydrofuryl oder 2-Tetrahydropyranyl oder entsprechende Thiaanaloge, sowie leicht abspaltbare, gegebenenfalls substituierte α-Phenylnieder-alkylreste, wie gegebenenfalls substituiertes Benzyl oder Diphenylmethyl, wobei als Substituenten der Phenylreste z.B. Halogen, wie Chlor, Niederalkoxy, wie Methoxy und/oder Nitro in Frage kommen.

Eine geschützte Sulfogruppe ist in erster Linie eine mit einem aliphatischen, cycloaliphatischen, cycloaliphatisch-aliphatischen, aromatischen oder araliphatischen Alkohol, wie einem Niederalkanol, oder mit einem Silyl- oder Stannylrest, wie Triniederalkylsilyl, veresterte Sulfogruppe. In einer Sulfogruppe kann die Hydroxygruppe beispielsweise wie die Hydroxygruppe in einer veresterten Carboxygruppe veräthert sein.

Der die Carboxylgruppe veresternde Rest $R_2$ ist bevorzugt unter schonenden Bedingungen, inklusive unter physiologischen Bedingungen, leicht abspaltbar. Solche Reste $R_2$ sind beispielsweise Niederalkyl, insbesondere tert.-Niederalkyl, z.B. tert.-Butylyl, Polycycloalkyl, z.B. Adamantyl, Arylmethyl, worin Aryl vorzugsweise einen oder zwei, gegebenenfalls, z.B. durch Niederalkyl, insbesondere tert.-Niederalkyl, z.B. tert.-Butyl, Niederalkoxy, wie Methoxy, Hydroxy, Halogen, z.B. Chlor, und/oder Nitro, mono- oder polysubstituierte Phenylreste darstellt, wie gegebenenfalls, z.B. wie oben erwähnt substituiertes Benzyl, z.B. 4-Nitro-benzyl, oder 4-Methoxybenzyl, oder z.B. wie oben erwähnt substituiertes Diphenylmethyl, z.B. Benzhydryl oder Di-(4-methoxyphenyl)-methyl, oder 2-Halogenniederalkyl, z.B. 2,2,2-Trichloräthyl, insbesondere Aroylmethyl, worin die Aroylgruppe vorzugsweise gegebenenfalls, z.B. durch Halogen, wie Brom, substituiertes Benzoyl darstellt, z.B. Phenacyl, oder Polyhalogenaryl, wie Pentachlorphenyl. $R_2$ ist ferner eine Silyl-, insbesondere eine organische Silylgruppe oder eine entsprechende Stannylgruppe. In diesen enthält das Silicium- bzw. Zinnatom vorzugsweise Niederalkyl, insbesondere Methyl, ferner Niederalkoxy, z.B. Methoxy, und/oder Halogen, z.B. Chlor, als Substituenten. Geeignete Silyl- bzw. Stannylschutzgruppen sind in erster Linie Triniederalkylsilyl, insbesondere Trimethylsilyl, ferner Dimethyl-tert.-butyl-silyl, Niederalkoxy-niederalkyl-halogen-silyl, z.B. Methoxy-methyl-chlor-silyl, oder Diniederalkyl-halogen-silyl, z.B. Dimethylchlor-silyl, oder entsprechend substituierte Stannylverbindungen, z.B. Tri-n-butyl-stannyl.

Bevorzugt als schützender Rest $R_2$ ist insbesondere tert.-Butyl, oder gegebenenfalls, z.B. wie erwähnt substituiertes, Benzyl, z.B. 4-Nitrobenzyl und Diphenylmethyl.

Ein unter physiologischen Bedingungen abspaltbarer Rest $R_2$ ist in erster Linie eine 1-Acyloxyniederalkylgruppe, worin Acyl z.B. den Rest einer organischen Carbonsäure, z.B. einer gegebenenfalls substituierten Niederalkancarbonsäure, oder der Acylrest eines Halbesters der Kohlensäure, bedeutet und Niederalkyl Methyl oder Aethyl darstellt, und worin Acyl und Niederalkyl durch eine C-C-Bindung miteinander verknüpft sein können. Solche Gruppen $R_2$ sind beispielsweise Niederalkanoyloxymethyl, wie Acetyloxymethyl, Pivaloyloxymethyl oder 1-Aethoxycarbonyloxy-aethyl, Aminoniederalkanoyloxymethyl, insbesondere α-Amino-niederalkanoyloxymethyl, z.B. Glycyloxymethyl, L-Valyloxymethyl oder L-Leucyloxymethyl, sowie 2-Phthalidyl.

Heterocyclylreste $R_3$ sind aromatischer Natur oder auch partiell gesättigt und gegebenenfalls substituiert. Solche heterocyclische Reste sind in erster Linie gegebenenfalls substituierte, z.B. die unten genannten Substituenten enthaltende, monocyclische, fünfgliedrige diaza-, triaza-, tetraza-, thiaza-, thiadiaza-, thiatriaza-,oxaza- oder oxadiazacyclische Reste aromatischen Charakters oder entsprechende, gegebenenfalls substituierte, z.B. die unten genannten Substituenten enthaltende Reste mit ankondensiertem Benzolring, wie benzodiaza- oder benzooxazacyclische Reste, gegebenenfalls die unten genannten Substituenten, in erster Linie Oxido enthaltende, monocyclische, sechsgliedrige monoaza- oder diazacyclische Reste aromatischen Charakters oder entsprechende, partiell gesättigte, gegebenenfalls substituierte, z.B. die unten genannten Substituenten, in erster Linie Oxo, enthaltende Reste, oder gegebenenfalls substituierte, z.B. die unten genannten Substituenten enthaltende, bicyclische triaza-, tetraza- oder pentazacyclische Reste aromatischen Charakters oder entsprechende partiell gesättigte, gegebenenfalls substituierte, z.B. die unten genannten Substituenten, in erster Linie Oxo, enthaltende Reste. Substituenten sind u.a. Niederalkyl, insbesondere Methyl, Hydroxy-niederalkyl, z.B. Hydroxymethyl, Carboxyniederalkyl, z.B. Carboxymethyl oder 1- oder 2-Carboxyäthyl, gegebenenfalls N-substituiertes Aminoniederalkyl, wie Diniederalkylaminoniederalkyl, z.B. Dimethylaminoäthyl, gegebenenfalls in Salzform vorliegendes Sulfoniederalkyl, z.B. gegebenenfalls als Natriumsalz vorliegendes Sulfomethyl oder 1- oder 2-Sulfoäthyl, Cycloalkyl, z.B. Cyclopentyl oder Cyclohexyl, Aryl, wie gegebenenfalls durch Halogen, z.B. Chlor, oder Nitro substituiertes Phenyl, Arylniederalkyl, z.B. Benzyl, oder funktionelle Gruppen, wie Halogen, z.B. Fluor, Chlor oder Brom, gegebenenfalls substituiertes Amino, wie gegebenenfalls durch Niederalkyl mono- oder disubstituiertes Amino, z.B. Amino, Methylamino oder Dimethylamino, Nitro, Hydroxy, Niederalkoxy, z.B. Methoxy oder Aethoxy, oder gegebenenfalls funktionell abgewandeltes Carboxyl, wie Carboxyl, verestertes Carboxy, wie Niederalkoxycarbonyl, z.B. Methoxycarbonyl oder Aethoxycarbonyl, gegebenenfalls substituiertes, wie N-mono- oder N,N-diniederalkyliertes Carbamoyl, z.B. N-Methylcarbamoyl oder N,N-Dimethylcarbamoyl, oder Cyan, sowie Oxo oder Oxido, wobei einer oder mehrere solcher Substituenten, die in erster Linie mit Ringkohlenstoffatomen, aber auch, insbesondere Niederalkyl und Oxido, mit Ringstickstoffatomen verbunden sind, vorhanden sein können. Beispiele solcher Heterocyclylreste sind Imidazolyl, z.B. 2-Imidazolyl, gegebenenfalls durch Niederalkyl und/oder Phenyl substituiertes Triazolyl, z.B. 1,2,3-Triazol-4-yl, 1-Methyl-1H-1,2,3-triazol-4-yl, 1H-1,2,4-Triazol-3-yl, 5-Methyl-1H-1,2,4-triazol-3-yl, 3-Methyl-1-phenyl-1H-1,2,4-triazol-5-yl, 4,5-Dimethyl-4H-1,2,4-triazol-3-yl oder 4-Phenyl-4H-1,2,4-triazol-3-yl, gegebenenfalls durch Niederalkyl, Carboxyniederalkyl, Sulfoniederalkyl oder Diniederalkylaminoniederalkyl, Phenyl oder Halogenphenyl substituiertes Tetrazolyl, z.B. 1H-Tetrazol-5-yl, 1-Methyl-1H-tetrazol-5-yl, 1-Phenyl-1H-tetrazol-5-yl, 1-(4-Chlorphenyl)-1H-tetrazol-5-yl, 1-Carboxymethyl-1H-tetrazol-5-yl, 1-(2-Carboxyäthyl)-1H-tetrazol-5-yl, 1-(2-Dimethylaminoäthyl)-1H-tetrazol-5-yl oder 1-Sulfomethyl-1H-tetrazol-5-yl, gegebenenfalls durch Niederalkyl oder Thienyl substituiertes Thiazolinyl, Thiazolyl oder Isothiazolyl, z.B. 2-Thiazolinyl, 2-Thiazolyl, 4-(2-Thienyl)-2-thiazolyl, 4,5-Dimethyl-2-thiazolyl, 3-Isothiazolyl, 4-Isothiazolyl oder 5-Isothiazolyl, gegebenenfalls durch Niederalkyl substituiertes Thiadiazolyl, z.B. 1,2,3-Thiadiazol-4-yl, 1,2,3-Thiadiazol-5-yl, 1,3,4-Thiadiazol-2-yl, 2-Methyl-1,3,4-thiadiazol-5-yl, 1,2,4-Thiadiazol-5-yl oder 1,2,5-Thiadiazol-3-yl, Thiatriazolyl, z.B. 1,2,3,4-Thiatriazolyl-5-yl, gegebenenfalls durch Niederalkyl oder Phenyl substituiertes Oxazolyl oder Isoxazolyl, z.B. 5-Oxazolyl, 4-Methyl-5-oxazolyl, 2-Oxazolyl, 4,5-Diphenyl-2-oxazolyl oder 3-Methyl-5-isoxazolyl, gegebenenfalls durch Niederalkyl, Sulfoniederalkyl, Phenyl, Nitrophenyl oder Thienyl substituiertes Oxadiazolyl, z.B. 1,2,4-Oxadiazol-5-yl, 2-Methyl-1,3,4-oxadiazol-5-yl, 2-Sulfomethyl-1,3,4-oxadiazol-5-yl, 2-Phenyl-1,3,4-oxadiazol-5-yl, 5-(4-Nitrophenyl)-1,3,4-oxadiazol-2-yl oder 2-(Thienyl)-1,3,4-oxadiazol-5-yl, gegebenenfalls durch Halogen substituiertes Benzimidazolyl, z.B. 2-Benzimidazolyl oder 5-Chlor-2-benzimidazolyl, oder gegebenenfalls durch Halogen oder Nitro substituiertes Benzoxazolyl, z.B. 2-Benzoxazolyl,

5-Nitro-2-benzoxazolyl oder 5-Chlor-2-benzoxazolyl, gegebenenfalls durch Halogen substituiertes 1-Oxido-pyridyl, z.B. 1-Oxido-2-pyridyl oder 4-Chlor-1-oxido-2-pyridyl, gegebenenfalls durch Hydroxy substituiertes Pyridazinyl, z.B. 3-Hydroxy-6-pyridazinyl, gegebenenfalls durch Niederalkyl, Niederalkoxy oder Halogen substituiertes N-Oxido-pyridazinyl, z.B. 2-Oxido-6-pyridazinyl, 3-Chlor-1-oxido-6-pyridazinyl, 3-Methyl-2-oxido-6-pyridazinyl, 3-Methoxy-1-oxido-6-pyridazinyl, 3-Aethoxy-1-oxido-6-pyridazinyl, 3-n-Butyloxy-1-oxido-6-pyridazinyl oder 3-(2-Aethylhexyloxy)-1-oxido-6-pyridazinyl, oder gegebenenfalls durch Niederalkyl, Amino, Diniederalkylamino oder Carboxy substituiertes 2-Oxo-1,2-dihydro-pyrimidinyl, z.B. 2-Oxo-1,2-dihydro-4-pyrimidinyl, 6-Methyl-2-oxo-1,2-dihydro-4-pyrimidinyl, 5-Methyl-2-oxo-1,2-dihydro-4-pyrimidinyl, 6-Amino-2-oxo-1,2-dihydro-4-pyrimidinyl, 6-Dimethylamino-2-oxo-1,2-dihydro-4-pyrimidinyl, 5-Carboxy-2-oxo-1,2-dihydro-4-pyrimidinyl oder 6-Carboxy-2-oxo-1,2-dihydro-4-pyrimidinyl, Triazolopyridyl, z.B. s-Triazolo[4,3-a]pyrid-3-yl oder 3H-v-Triazolo[4,5-b]pyrid-5-yl, gegebenenfalls durch Halogen und/oder Niederalkyl substituiertes Purinyl, z.B. 2-Purinyl, 6-Purinyl oder 8-Chlor-2-methyl-6-purinyl, ferner 2-Oxo-1,2-dihydro-purinyl, z.B. 2-Oxo-1,2-dihydro-6-purinyl, oder Tetrazolopyridazinyl, z.B. Tetrazolo[4,5-b]pyridazin-6-yl oder Tetrazolo[4,5-b]pyridazin-7-yl.

Salze sind insbesondere diejenigen von Verbindungen der Formel I mit einer freien Carboxylgruppe, in erster Linie Metall- oder Ammoniumsalze, wie Alkalimetall- und Erdalkalimetall-, z.B. Natrium-, Kalium-, Magnesium- oder Calciumsalze, sowie Ammoniumsalze mit Ammoniak oder geeigneten organischen Aminen, wobei in erster Linie aliphatische, cycloaliphatische, cycloaliphatisch-aliphatische oder araliphatische primäre, sekundäre oder tertiäre Mono-, Di- oder Polyamine, sowie heterocyclische Basen für die Salzbildung in Frage kommen, wie Niederalkylamine, z.B. Triäthylamin, Hydroxyniederalkylamine, z.B. 2-Hydroxyäthylamin, Bis-(2-hydroxyäthyl)-amin oder Tris-(2-hydroxyäthyl)-amin, sowie basische aliphatische Ester von Carbonsäuren, z.B. 4-Aminobenzoesäure-2-diäthylaminoäthylester, Niederakylenamine, z.B. 1-Aethylpiperidin, Cycloalkylamine, z.B. Dicyclohexylamin, oder Benzylamine, z.B. N,N'-Dibenzyl-äthylendiamin, ferner Basen vom Pyridintyp, z.B. Pyridin, Collidin oder Chinolin. Verbindungen der Formel I mit einer basischen Gruppe können Säureadditionssalze, z.B. mit anorganischen Säuren, wie Salzsäure, Schwefelsäure oder Phosphorsäure, oder mit geeigneten organischen Carbon- oder Sulfonsäuren, z.B. Trifluoressigsäure, sowie mit Aminosäuren, wie Arginin und Lysin bilden. Verbindungen der Formel I mit einer freien Carboxylgruppe und freien Aminogruppe können auch in Form von inneren Salzen, d.h. in zwitterionischer Form vorliegen.

Das gegebenenfalls im Rest A vorhandene Asymmetrizentrum, nämlich wenn A Hydroxymethylen, Aminomethylen oder Sulfomethylen bedeutet, liegt in der R,S- oder bevorzugt in der R-Konfiguration vor. Hydroxyimino- und Methoxyiminomethylengruppen A liegen bevorzugt in der syn-Form (Z-Form) vor.

Die Verbindungen der Formel I, worin die Carboxylgruppe gegebenenfalls in physiologisch spaltbarer Form verestert ist und die funktionellen Gruppen im Rest A in ungeschützter Form vorliegen, und ihre pharmazeutisch verwendbaren, nichttoxischen Salze sind wertvolle, antibiotisch wirksame Substanzen, die insbesondere als antibakterielle Antibiotika verwendet werden können.

Beispielsweise sind sie in vitro gegen grampositive und gramnegative Mikroorganismen, wie gegen Kokken, z.B. Staphylokokken, wie Staphylococcus aureus, Streptokokken, wie Streptococcus faecalis und Streptococcus pneumoniae, Enterokokken und Mikrokokken, inklusive Neisseria Arten, z.B. Neisseria gonorrhoeae, in Minimalkonzentrationen von etwa 0,005 mcg/ml bis etwa 32 mcg/ml, und gegen gramnegative Stäbchenbakterien, wie Enterobacteriaceae, sowie gegen Pseudomonas aeruginosa und Haemophilus influenzae in Minimalkonzentration von etwa 0,005 bis etwa 32 mcg/ml, wirksam. In vivo, bei subcutaner Application an der Maus, sind sie ebenfalls gegen grampositive und gramnegative Erreger wie, gegen grampositive Kokken, z.B. Staphylococcus aureus, in Minimaldosen von etwa 8 bis etwa 100 mg/kg, und gegen Enterobakterien, z.B. Escherichia coli, Proteus morganii etc., in Minimaldosen von etwa 2 bis etwa 100 mg/kg wirksam. Die neuen Verbindungen können deshalb entsprechend, z.B. in Form von antibiotisch wirksamen Präparaten, zur Behandlung von durch grampositive oder gramnegative Infektionserreger verursachten Infektionen Verwendung finden.

Verbindungen der Formel (I), worin die funktionellen Gruppen geschützt sind, werden als Ausgangsmaterialien zur Herstellung von antibiotisch wirksamen Verbindungen der Formel I verwendet.

Die vorliegende Erfindung betrifft in erster Linie diejenigen Verbindungen der Formel I, worin $R_1$ Wasserstoff, Niederalkyl, Niederalkanoyl oder 2-Halogenniederalkanoyl bedeutet, A Methylen, Aminomethylen, Hydroxymethylen, Sulfomethylen, Hydroximinomethylen oder Methoxyiminomethylen darstellt, $R_2$ Wasserstoff und $R_3$ eine der genannten Heterocyclylgruppen, insbesondere eine der genannten gegebenenfalls substituierten Triazolyl, Tetrazolyl-, Thiazolinyl,

Thiadiazolyl-, Oxadiazolyl-, Pyridazinyl- oder Tetrazolopyridazinylgruppen, z.B. 1,2,3-Triazol-4-yl, 1-Methyl-1H-tetrazol-5-yl, 1-Carboxymethyl-1H-tetrazol-5-yl, 1-(2-Carboxyäthyl)-1H-tetrazol-5-yl, 1-(2-Dimethylaminoäthyl)-1H-tetrazol-5-yl, 1-Sulfomethyl-1H-tetrazol-5-yl, Thiazolin-2-yl, 1,3,4-Thiadiazol-5-yl, 2-Methyl-1,3,4-thiadiazol-5-yl, 2-Sulfomethyl-1,3,4-oxydiazol-5-yl, 3-Hydroxy-6-pyridazinyl, 3-Methyl-2-oxido-6-pyridazinyl, oder Tetrazolo[4,5-b]pyridazin-6-yl, bedeutet, ihre physiologisch spaltbaren Ester, z.B. Pivaloyloxymethylester, und ihre Salze, wie pharmazeutisch annehmbaren Salze, sowie die entsprechenden Verbindungen mit geschützten funktionellen Gruppen.

Besonders hervorzuheben sind Verbindungen der Formel (I), worin $R_1$ insbesondere Wasserstoff, sowie 2-Halogenniederalkanoyl, wie 2-Chlorniederalkanoyl, bedeutet, A Hydroxyiminomethylen oder Methoxyiminomethylen darstellt, $R_2$ Wasserstoff und $R_3$ eine der genannten Tetrazolylgruppen, insbesondere 1-Methyl-1H-tetrazol-5-yl, 1-Carboxymethyl-1H-tetrazol-5-yl, 1-(2-Carboxyäthyl)-1H-tetrazol-5-yl, 1-(2-Dimethylaminoäthyl)-1H-tetrazol-5-yl, 1-Sulfomethyl-1H-tetrazol-5-yl, oder Thiazolin-2-yl, 1,3,4-Thiadiazol-5-yl oder 2-Methyl-1,3,4-thiadiazol-5-yl, oder 1,2,3-Triazol-4-yl bedeutet, ihre physioligisch spaltbaren Ester, z.B. Pivaloyloxymethylester, und ihre Salze, wie pharmazeutisch annehmbaren Salze, sowie die entsprechenden Verbindungen mit geschützten funktionellen Gruppen.

Die Erfindung betrifft insbesondere die in den Beispielen beschriebenen Verbindungen der Formel I, deren Salze, wie pharmazeutisch annehmbare Salze, sowie die dort beschriebenen neuen Ausgangsstoffe und Zwischenprodukte.

Die neuen Verbindungen können in an sich bekannter Weise erhalten werden.

So werden Verbindungen der Formel I hergestellt, indem man

a) die 7ß-Amninogruppe in einer Verbindung der Formel

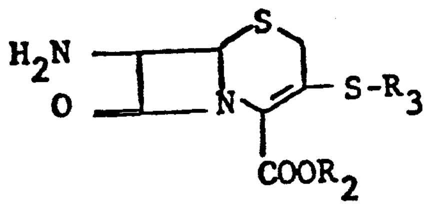

(II),

worin die Aminogruppe gegebenenfalls durch eine die Acylierung erlaubende Gruppe substituiert ist, und worin $R_2$ Wasserstoff oder einen die Carboxylgruppe veresternden Rest darstellt, durch Behandeln mit einem den Acylrest einer Carbonsäure der Formel

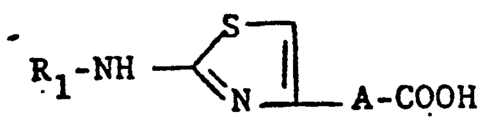

(III)

einführenden Acylierungsmittel,
worin gegebenenfalls vorhandene funktionelle Gruppe in geschützter Form vorliegen, acyliert, oder

b) eine Verbindung der Formel

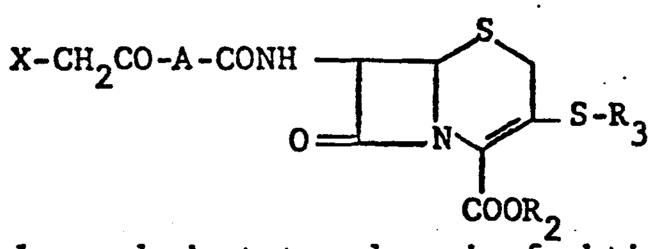

(IV),

worin X Halogen bedeutet, und worin funktionelle Gruppen gegebenenfalls geschützt sind, oder ein Salz davon, mit einem Thioharnstoff der Formel $R_1$-NH-CS-$NH_2$ oder einem Salz davon kondensiert, oder

c) eine Verbindung der Formel

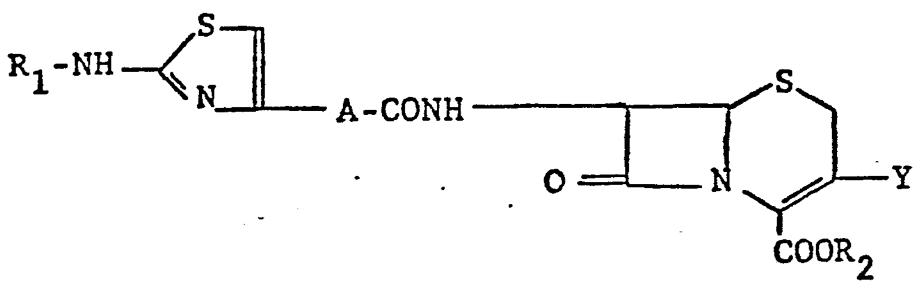

(V),

worin Y eine gegen den Thiolrest $-S-R_3$ austauschbare Gruppe darstellt, und worin funktionelle Gruppen gegebenenfalls geschützt sind, in Gegenwart einer Base mit einem Thiol der Formel $HS-R_3$, wobei in $R_3$ vorhandene funktionelle Gruppen gegebenenfalls geschützt sind, und gegebenenfalls mit einer Säure behandelt, oder

d) eine isomere Verbindung der Formel

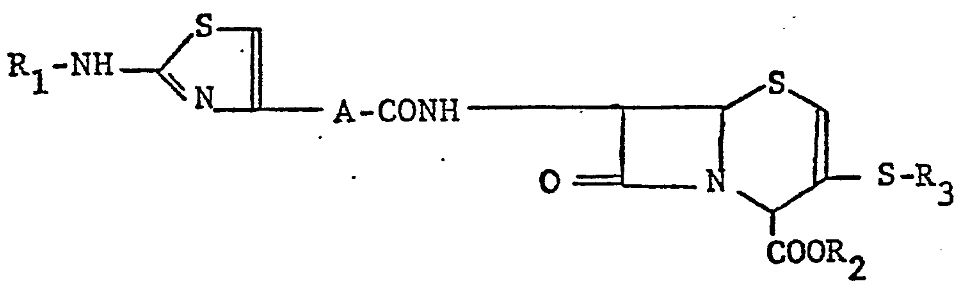

(VI),

isomerisiert, und, wenn erwünscht, in einer erhaltenen Verbindung einen Rest $R_1$, $R_2$, $R_3$ oder A in einen anderen Rest $R_1$, $R_2$, $R_3$ bzw. A überführt, und/oder, wenn erwünscht, eine erhaltene Verbindung in ein Salz oder ein erhaltenes Salz in eine freie Verbindung oder in ein anderes Salz überführt.

Verfahren a): Gegebenenfalls vorhandene, die Aminogruppe substituierende und deren Acylierung erlaubende Reste in einem Ausgangsmaterial der Formel II sind beispielsweise organische Silyl- oder Stannylgruppen, ferner auch Ylidengruppen, die zusammen mit der Aminogruppe eine Schiff'sche Base bilden. Die genannten organischen Silyl- oder Stannylgruppen sind z.B. die gleichen

die auch mit der 4-Carboxylgruppe am Cephemring eine geschützte Carboxylgruppe zu bilden vermögen. Bei der Silylierung oder Stannylierung einer Carboxylgruppe in einem Ausgangsmaterial der Formel II,kann, bei Verwendung eines Ueberschusses des Silylierungs- oder Stannylierungsmittels, die Aminogruppe ebenfalls silyliert oder stannyliert werden.

Die genannten Ylidengruppen sind in erster Linie Arylmethylengruppen, worin Aryl insbesondere für einen carbocyclischen, in erster Linie monocyclischen Arylrest, z.B. für gegebenenfalls, wie durch Nitro oder Hydroxy, substituiertes Phenyl steht;solche Arylmethylengruppen sind z.B. Benzyliden, 2-Hydroxybenzyliden oder 4-Nitrobenzyliden, ferner gegebenenfalls, z.B. durch Carboxy substituiertes Oxacycloalkyliden, z.B. 3-Carboxy-2-oxacyclohexyliden.

Den Acylrest einer Carbonsäure der Formel (III) einführende Acylierungsmittel sind beispielsweise die Carbonsäure selbst oder reaktionsfähige funktionelle Derivate davon.

Falls eine freie Säure der Formel III, worin gegebenenfalls alle funktionellen Gruppen ausser der reagierenden Carboxylgruppe geschützt sind, als Acylierungsmittel eingesetzt wird, verwendet man üblicherweise geeignete Kondensationsmittel, wie Carbodiimide, beispielsweise N,N'-Diäthyl-, N,N'-Dipropyl-, N,N'-Diisopropyl-, N,N'-Dicyclohexyl- oder N-Aethyl-N'-3-dimethylaminopropyl-carbodiimid, geeignete Carbonylverbindungen, beispielsweise Carbonyldiimidazol, oder Isoxazoliniumsalze, beispielsweise N-Aethyl-5-phenyl-isoxazolinium-3'-sulfonat und N-tert.-Butyl-5-methyl-isoxazoliniumperchlorat, oder eine Acylaminoverbindung, z.B.

2-Aethoxy-1-äthoxycarbonyl-1,2-dihydro-chinolin.

Die Kondensationsreaktion wird vorzugsweise in einem wasserfreien Reaktionsmedium, vorzugsweise in Gegenwart eines Lösungs- oder Verdünnungsmittels, z.B. Methylenchlorid, Dimethylformamid, Acetonitril oder Tetrahydrofuran, wenn erwünscht oder notwendig, unter Kühlen oder Erwärmen und/oder in einer Inertgasatmosphäre, durchgeführt.

Ein reaktionsfähiges, d.h. Amid-bildendes, funktionelles Derivat einer Säure der Formel III, worin alle übrigen funktionellen Gruppen gegebenenfalls geschützt sind, ist in erster Linie ein Anhydrid einer solchen Säure, inklusive und vorzugsweise ein gemischtes Anhydrid, aber auch ein inneres Anhydrid, d.h. ein entsprechendes Keten. Gemischte Anhydride sind z.B. diejenigen mit anorganischen Säuren, wie Halogenwasserstoffsäuren, d.h. die entsprechenden Säurehalogenide, z.B. -chloride oder -bromide, ferner mit Stickstoffwasserstoffsäure, d.h. die entsprechenden Säureazide, mit einer phosphorhaltigen Säure, z.B. Phosphor-, Phosphon- oder Phosphinsäure, z.B. der Formel $(R^1)(R^2)P(=O)-OH$, worin $R^1$ und $R^2$ die oben angegebenen Bedeutungen haben, und insbesondere Aethoxy bedeuten, oder mit einer schwefelhaltigen Säure, z.B. Schwefelsäure, oder mit Cyanwasserstoffsäure. Weitere gemischte Anhydride sind z.B. diejenigen mit organischen Carbonsäuren, wie mit gegebenenfalls, z.B. durch Halogen, wie Fluor oder Chlor, substituierten Niederalkancarbonsäuren, z.B. Pivalinsäure oder Trichloressigsäure, oder mit Halbestern, insbesondere Niederalkylhalbestern der Kohlensäure, wie dem Aethyl- oder Isobutylhalbester der Kohlensäure, oder mit organischen, insbesondere aliphatischen oder aromatischen, Sulfonsäuren, z.B. p-Toluolsulfonsäure. Von der Säure III,

wenn A Hydroxymethylen ist, kann auch ein gemischtes inneres Anhydrid, z.B. mit dem Kohlensäurehalbester der α-Hydroxygruppe verwendet werden.

Weitere reaktionsfähige Säurederivate einer Säure der Formel III, sind aktivierte Ester, wie Ester mit vinylogen Alkoholen (d.h. Enolen), wie vinylogen Niederalkenolen, oder Arylester, wie 4-Nitrophenyl- oder Pentachlorphenylester, heteroaromatische Ester, wie Benztriazolyl-, z.B. 1-Benztriazolylester, oder Diacyliminoester, wie Succinylimino- oder Phthalyliminoester.

Die Acylierung mit einem Säurederivat, wie einem Anhydrid, insbesondere mit einem Säurehalogenid, wird bevorzugt in Anwesenheit eines säurebindenden Mittels, beispielsweise einer organischen Base, wie eines organischen Amins, z.B. eines tertiären Amins, wie Triniederalkylamin, z.B. Trimethylamin, Triäthylamin oder Aethyl-diisopropylamin, oder N,N-Diniederalkyl-anilin, z.B. N,N-Dimethylanilin, oder eines cyclischen tertiären Amins, wie eines N-niederalkylierten Morpholins, wie N-Methylmorpholin, oder einer Base vom Pyridin-Typ, z.B. Pyridin, einer anorganischen Base, beispielsweise eines Alkalimetall- oder Erdalkalimetallhydroxids, -carbonats oder -hydrogencarbonats, z.B. Natrium-, Kalium- oder Calciumhydroxid, -carbonat oder -hydrogencarbonat, oder eines Oxirans, beispielsweise eines niederen 1,2-Alkylenoxids, wie Aethylenoxid oder Propylenoxid, durchgeführt.

Die obigen Acylierungen werden bevorzugt in einem inerten, vorzugsweise wasserfreien Lösungsmittel oder Lösungsmittelgemisch vorgenommen, beispielsweise in einem Carbonsäureamid, wie einem Formamid, z.B. Dimethylformamid, einem halogenierten Kohlenwasserstoff, z.B. Methylenchlorid, Tetrachlorkohlenstoff oder Chlorbenzol, einem Keton, z.B. Aceton, einem Ester, z.B.

Essigsäureäthylester, oder einem Nitril, z.B. Acetonitril, oder Mischungen davon, bei Raumtemperatur, wenn notwendig, bei erniedrigter oder erhöhter Temperatur, etwa bei -40° bis etwa 100°, bevorzugt bei -10° bis +40°, und/oder in einer Inertgas-, z.B. Stickstoffatmosphäre.

Die Acylierung kann auch in Gegenwart einer die gewünschte Amidbindung herstellenden Acylase erfolgen. Solche Acylasen sind bekannt und werden durch eine Reihe von Mikroorganismen gebildet, z.B. durch Acetobacter, wie Acetobacter aurantium, Achromobacter, wie Achromobacter aeris, Aeromonas, wie Aeromonas hydrophila, oder Bacillus megaterium, z.B. 400. In die enzymatische Acylierung werden insbesondere Amide, Ester oder Thioester, wie entsprechende Niederalkyl-, z.B. Methyl- oder Aethylester, der Carbonsäure der Formel III eingesetzt. Die enzymatische Acylierung wird in einem den Mikroorganismus enthaltenden Nährmedium, in einem Filtrat der Kulturbrühe oder, gegebenenfalls nach Isolierung der Acylase, inklusive nach Adsorption an einen Träger, in einem wässrigen, gegebenenfalls einen Puffer enthaltenden Medium bei etwa 30-40°, bevorzugt bei etwa 37°, durchgeführt.

In einer acylierenden Säure der Formel III oder in einem Säurederivat davon kann eine geschützte Aminogruppe auch in ionischer Form vorliegen, d.h. das Ausgangsmaterial der Formel III kann in Form eines Säureadditionssalzes, vorzugsweise mit einer starken anorganischen Säure, wie einer Halogenwasserstoffsäure, z.B. Salzsäure, oder Schwefelsäure verwendet werden.

Ein Säurederivat kann, wenn erwünscht, <u>in situ</u> gebildet werden. So erhält man z.B. ein gemischtes Anhydrid durch Behandeln einer Säure der Formel III mit gegebenenfalls entsprechend geschützten funktionellen Gruppen, oder eines geeigneten Salzes davon, wie eines

Ammoniumsalzes, z.B. mit einem organischen Amin, wie 4-Methylmorpholin oder Pyridin, oder eines Metall-, z.B. Alkalimetallsalzes, mit einem geeigneten Säurederivat, wie einem entsprechenden Säurehalogenid, z.B. einer der genannten Phosphor-, Phosphon- oder Phosphinsäuren, wie Diäthylphosphorobromidat, oder einer gegebenenfalls substituierten Niederalkancarbonsäure, z.B. Trichloracetylchlorid, oder mit einem Halbester eines Kohlensäurehalbhalogenids, z.B. Chlorameisensäureäthylester oder -isobutylester, wobei gegebenenfalls noch vorhandene freie funktionelle Gruppen, z.B. Amino oder Hydroxy, durch den gleichen Acylrest geschützt werden können, und verwendet das so erhältliche gemischte Anhydrid ohne Isolierung.

Verfahren b): In einer Ausgangsverbindung der Formel IV is X als Halogen, insbesondere Chlor, aber auch Brom, Jod oder Fluor. Der Thioharnstoff wird in freier Form oder als Salz,insbesondere als Thiolat eines Alkalimetalls, wie Lithium, Natrium oder Kalium, oder auch als Thiolat einer Ammoniumverbindung, in äquivalenter Menge oder im Ueberschuss, eingesetzt. Eine Verbindung der Formel IV, die saure Gruppen enthält, z.B. wenn $R_2$ Wasserstoff ist oder wenn A Sulfomethylen bedeutet, kann ebenfalls als Salz, z.B. als Alkalimetall- oder als Ammoniumsalz, z.B. als Lithium-, Natrium-, Kalium-, Triniederalkyl-, wie Trimethyl- oder Triäthylammoniumsalz, eingesetzt werden.

Die Reaktion wird im allgemeinen in einem Lösungsmittel, wie Wasser oder einem organischen, inerten Lösungsmittel oder einer Mischung davon, durchgeführt. Als organische Lösungsmittel geeignet sind Alkohole, wie Methanol, Aethanol oder Isopropanol, Ketone, wie Aceton, Aether, wie Dioxan oder Tetrahydrofuran, Nitrile, wie Acetonitril, halogenierte Kohlenwasserstoffe, wie

Methylenchlorid, Chloroform oder Tetrachlorkohlenstoff, Ester, wie Aethylacetat, oder Amide, wie Dimethylformamid oder Dimethylacetamid, und ähnliche. Die Reaktion kann, falls die freien Verbindungen eingesetzt werden, in Gegenwart einer Base durchgeführt werden. Geeignete Basen sind Alkalimetallhydroxide, wie Natrium- oder Kaliumhydroxid, Alkalimetallcarbonate, wie Natrium- oder Kaliumcarbonat, oder organische tertiäre Stickstoffbasen, wie Triniederalkylamine, z.B. Trimethylamin, Triäthylamin, Aethyl-diisopropylamin, Pyridin und dergleichen.

Die Reaktionstemperatur liegt bei Raumtemperatur, oder auch tiefer oder höher, vorzugsweise zwischen -10 bis +100° C, insbesondere zwischen 0 bis 40° C.

Die Reaktion kann auch stufenweise erfolgen, indem zunächst das ringoffene Zwischenprodukt mit der Teilformel $R_1$-NH-C(=NH)-S-$CH_2$-CO-A- entsteht, das dann in der zweiten Stufe dehydratisiert wird.

Verfahren c): In einer Verbindung der Formel (V) ist die austauschbare Gruppe Y insbesondere eine veresterte oder verätherte Hydroxygruppe. Eine veresterte Hydroxygruppe Y ist durch eine anorganische oder organische Säure verestert, beispielsweise durch eine Halogenwasserstoffsäure, eine Schwefel oder Phosphor enthaltende Säure, wie eine Schwefel- oder Sulfonsäure, eine Phosphor-, Phosphon- oder Phosphinsäure, oder eine Carbonsäure. Entsprechend ist Y beispielsweise Halogen, insbesondere Chlor, Brom oder Jod, eine Gruppe $-O-SO_2R^1$ oder $-O-P(=O)(R^1)(R^2)$, worin $R^1$ und $R^2$ die oben angegebenen Bedeutungen haben, oder eine Gruppe $-O-Ac^o$, worin $Ac^o$ beispielsweise der Acylrest einer organischen Carbonsäure mit z.B. bis zu 18 C-Atomen, insbesondere einer der oben unter Ac genannten gegebenenfalls, z.B. durch Halogen oder Aryl, substituierten aliphati-

schen Carbonsäure oder einer gegebenenfalls, z.B. durch Halogen, Niederalkoxy oder Nitro, substituierten aromatischen Carbonsäure. Eine veräthertе Hydroxygruppe Y ist durch einen Kohlenwasserstoffrest veräthert, beispielsweise einen aliphatischen, cycloaliphatischen, aromatischen oder araliphatischen Kohlenwasserstoffrest mit z.B. bis zu 15 C-Atomen, wie $C_1$-$C_4$-Niederalkyl, z.B. Methyl oder Aethyl, $C_2$-$C_6$ Niederalkenyl, z.B. Vinyl oder Propenyl, $C_3$-$C_7$-Cycloalkyl, z.B. Cyclopropyl oder Cyclohexyl, Phenyl, oder Phenylniederalkyl, wie Benzyl.

Bevorzugt ist Y Chlor, Brom, Niederalkansulfonyloxy, wie Methan- oder Aethansulfonyloxy, oder Arylsulfonyloxy, wie Benzol-, p-Toluol-, p-Chlorbenzol- oder p-Nitrobenzolsulfonyloxy.

Die Substitution der Gruppe Y durch die Gruppe -S-$R_3$ findet in an sich bekannter Weise unter Kühlen, bei Raumtemperatur oder unter Erwärmen, d.h. bei Temperaturen zwischen etwa -70° C und etwa + 100° C, bevorzugt bei etwa 0° C bis etwa 40° C, in einem geeigneten inerten Lösungsmittel und in Gegenwart einer Base statt. Geeignete inerte Lösungsmittel sind beispielsweise aliphatische, cycloaliphatische oder aromatische, gegebenenfalls substituierte, z.B. halogenierte, wie fluorierte oder chlorierte, Kohlenwasserstoffe, wie Hexan, Cyclohexan, Benzol, Toluol, Xylol, Methylenchlorid, Chloroform oder Tetrachlorkohlenstoff, ätherartige Lösungsmittel, wie Diniederalkyläther, z.B. Diäthyläther, Diisopropyläther, 1,2-Dimethoxyäthan, Tetrahydrofuran oder Dioxan, N,N-Diniederalkylamide, wie N,N-Dimethylformamid oder N,N-Dimethylacetamid, Niederalkylnitrile, wie Acetonitril, Diniederalkylsulfide, wie Dimethylsulfid, Diniederalkylsulfoxide, wie Dimethylsulfoxid oder Gemische davon. Geeignete Basen sind insbesondere sterisch gehinderte Amine, wie Triniederalkylamine, z.B. N,N-Diisopropyl-N-äthylamin, bicyclische Amidine, wie Diazabicycloalkane, z.B. 1,5-Diazabicyclo[4.3.0]non-5-en oder 1,5-Diazabicyclo[5.4.0]undec-5-en, sowie Alkalimetallhydride, -amide oder -niederalkanolate, wie Natriumhydrid, Natrium- oder Lithiumamid, Natriumäthylat oder Kalium-tert.-butylat. Die Reaktion kann in einer Inertgas-, wie Stickstoffatmosphäre durchgeführt werden, und, wenn notwendig, in einem geschlossenen Gefäss unter Druck.

Falls Y im Ausgangsmaterial der Formel (V) eine verätherte Hydroxygruppe ist, findet unter den basischen Bedingungen zunächst eine Addition des Thiols HS-$R_3$ an die Doppelbindung statt, worauf der Alkoholrest H-Y abgespalten werden muss.

Die Abspaltung des Alkohols H-Y findet in Gegenwart einer Säure, in An- oder Abwesenheit eines geeigneten, inerten Lösungsmittels und unter Kühlen, bei Raumtemperatur oder unter Erwärmen, d.h. bei Temperaturen zwischen etwa -70° C und +100° C, bevorzugt bei etwa 5° C bis etwa 40° C, statt. Zur Abspaltung von H-Y geeignete Säuren sind starke organische Protonensäuren, insbesondere Mineralsäuren, wie Salzsäure, Schwefelsäure, Salpetersäure oder Phosphorsäure, ferner Sulfonsäuren, wie Niederalkansulfonsäuren, z.B. Methansulfonsäure, oder aromatische Sulfonsäuren, z.B. Benzol- oder Toluolsulfonsäure, halogenierte Niederalkancarbonsäuren wie Trifluor- oder Trichloressigsäure oder auch Ameisensäure. Geeignete Lösungsmittel sind die bei der basischen Addition genannten.

Bei den vorstehenden Verfahren können, insbesondere wenn unter basischen Bedingungen gearbeitet wird, Gemische von 2-Cephemverbindungen der Formel VI und 3-Cephemverbindungen der Formel I entstehen. Durch übliche

Trennmethoden, insbesondere durch Chromatographie und Umkristallisation, können aus den Gemischen die reinen Isomeren erhalten werden.

Verfahren d): In einer 2-Cephem-Verbindung der Formel (VI) weist die gegebenenfalls geschützte Carboxylgruppe in 4-Stellung vorzugsweise die α-Konfiguration auf.

2-Cephem-Verbindungen der Formel (VI) werden isomerisiert, indem man sie mit einem schwach-basischen Mittel behandelt und die entsprechende 3-Cephem-Verbindung isoliert. Geeignete Isomerisierungsmittel sind z.B. organische, stickstoffhaltige Basen, insbesondere tertiäre heterocyclische Basen aromatischen Charakters, in erster Linie Basen des Pyridin-Typs, wie Pyridin selber, sowie Collidine oder Lutidine, ferner Chinolin, tertiäre aromatische Basen, z.B. solche des Anilin-Typs, wie N,N-Diniederalkylaniline, z.B. N,N-Dimethylanilin oder N,N-Diäthylanilin, oder tertiäre aliphatische, azacycloaliphatische oder araliphatische Basen, wie N,N,N-Triniederalkylamine, z.B. N,N,N-Trimethylamin, N,N-Dimethyl-N-äthylamin, N,N,N-Triäthylamin oder N,N,N-Diisopropyl-N-äthylamin, N-Niederalkyl-azacycloalkane, z.B. N-Methyl-piperidin, oder N-Phenyl-niederalkyl-N,N-diniederalkyl-amine, z.B. N-Benzyl-N,N-dimethylamin, sowie Gemische davon, wie das Gemisch einer Base vom Pyridintyp und eines N,N,N-Tri-niederalkylamins, z.B. Pyridin und Triäthylamin. Ferner können auch anorganische oder organische Salze von Basen, insbesondere von mittelstarken bis starken Basen mit schwachen Säuren, wie Alkalimetall- oder Ammoniumsalze von Niederalkancarbonsäuren, z.B. Natriumacetat, Triäthylammoniumacetat oder N-Methyl-piperidinacetat, sowie andere analoge Basen oder Gemische von solchen basischen Mitteln verwendet werden.

Bei Isomerisierung mit basischen Mitteln arbeitet man vorzugsweise in wasserfreiem Medium,in An- oder Abwesenheit eines Lösungsmittels,wie eines gegebenenfalls halogenierten, z.B. chlorierten, aliphatischen, cycloaliphatischen oder aromatischen Kohlenwasserstoffs, oder eines Lösungsmittelgemisches, wobei als Reaktionsmittel verwendete, unter den Reaktionsbedingungen flüssige Basen gleichzeitig auch als Lösungsmittel dienen können, unter Kühlen, bei Zimmertemperatur oder unter Erhitzen, vorzugsweise in einem Temperaturbereich von etwa -30°C bis etwa +100°C, in einer Inertgas-, z.B. Stickstoffatmosphäre, und/oder in einem geschlossenen Gefäss.

Die so erhältlichen 3-Cephem-verbindungen lassen sich in an sich bekannter Weise, z.B. durch Adsorption und/oder Kristallisation, von gegebenenfalls noch vorhandenen 2-Cephem-verbindungen abtrennen.

Die Isomerisierung von 2-Cephem-verbindungen der Formel (VI) wird bevorzugt durchgeführt, indem man diese in 1-Stellung oxidiert, wenn erwünscht, ein erhältliches Isomerengemisch der 1-Oxide trennt, und die so erhältliches 1-Oxyde der entsprechenden 3-Cephem-verbindungen reduziert.

Als geeignete Oxidationsmittel für die Oxidation in 1-Stellung von 2-Cephem-verbindungen kommen anorganische Persäuren, die ein Reduktionspotential von wenigstens +1,5 Volt aufweisen und aus nicht-metallischen Elementen bestehen, organische Persäuren oder Gemische aus Wasserstoffperoxid und Säuren, insbesondere organische Carbonsäuren, mit einer Dissoziationskonstante von wenigstens $10^{-5}$ in Frage. Geeignete anorganische Persäuren sind Perjod- und Perschwefelsäure. Organische Persäuren sind entsprechende Percarbon- und Persulfonsäuren, die

als solche zugesetzt oder durch Verwendung von wenigstens einem Aequivalent Wasserstoffperoxyd und einer Carbonsäure *in situ* gebildet werden können. Dabei ist es zweckmässig, einen grossen Ueberschuss der Carbonsäure zu verwenden, wenn z.B. Essigsäure als Lösungsmittel verwendet wird. Geeignete Persäuren sind z.B. Perameisensäure, Peressigsäure, Trifluorperessigsäure, Permaleinsäure, Perbenzoesäure, 3-Chlorperbenzoesäure,Monoperphthalsäure oder p-Toluolpersulfonsäure.

Die Oxidation kann ebenfalls unter Verwendung von Wasserstoffperoxid mit katalytischen Mengen einer Säure mit einer Dissoziationskonstante von wenigstens $10^{-5}$ durchgeführt werden, wobei man niedrige Konzentrationen, z.B. 1-2% und weniger, aber auch grössere Mengen der Säure einsetzen kann. Dabei hängt die Wirksamkeit des Gemisches in erster Linie von der Stärke der Säure ab. Geeignete Gemische sind z.B. solche von Wasserstoffperoxid mit Essigsäure, Perchlorsäure oder Trifluoressigsäure.

Die obige Oxidation kann in Gegenwart von geeigneten Katalysatoren durchgeführt werden. So kann z.B. die Oxidation mit Percarbonsäuren durch die Anwesenheit einer Säure mit einer Dissoziationskonstante von wenigstens $10^{-5}$ katalysiert werden, wobei ihre Wirksamkeit von ihrer Stärke abhängt. Als Katalysatoren geeignete Säuren sind z.B. Essigsäure, Perchlorsäure und Trifluoressigsäure. Ueblicherweise verwendet man mindestens äquimolare Mengen des Oxidationsmittels, vorzugsweise einen geringen Ueberschuss von etwa 10% bis etwa 20%, wobei man auch grössere Ueberschüsse, d.h. bis zur 10-fachen Menge des Oxidationsmittels oder darüber, verwenden kann. Die Oxidation wird unter milden Bedingungen, z.B. bei Temperaturen von etwa -50°C bis etwa +100°C, vorzugsweise von etwa -10°C bis etwa +40°C durchgeführt.

Die Reduktion der 1-Oxide von 3-Cephem-Verbindungen kann in an sich bekannter Weise durch Behandeln mit einem Reduktionsmittel, wenn notwendig, in Anwesenheit eines aktivierenden Mittels, durchgeführt werden. Als Reduktionsmittel kommen beispielsweise in Betracht: Katalytisch aktivierter Wasserstoff, wobei Edelmetallkatalysatoren verwendet werden, welche Palladium, Platin oder Rhodium enthalten, und die man gegebenenfalls zusammen mit einem geeigneten Trägermaterial, wie Kohle oder Bariumsulfat, einsetzt; reduzierende Zinn-, Eisen-, Kupfer- oder Mangankationen, welche in Form von entsprechenden Verbindungen oder Komplexen anorganischer oder organischer Art, z.B. als Zinn-II-chlorid, -fluorid, -acetat oder -formiat, Eisen-II-chlorid, -sulfat, -oxalat oder -succinat, Kupfer-I-chlorid, -benzoat oder -oxyd, oder Mangan-II-chlorid, -sulfat, -acetat oder -oxyd, oder als Komplexe, z.B. mit Aethylendiamintetraessigsäure oder Nitrolotriessigsäure, verwendet werden; reduzierende Dithionit-, Jod- oder Eisen-II-cyanid-anionen, welche in Form von entsprechenden anorganischen oder organischen Salzen, wie Alkalimetall-, z.B. Natrium- oder Kaliumdithionit, Natrium- oder Kaliumjodid oder -eisen-II-cyanid, oder in Form der entsprechenden Säuren, wie Jodwasserstoffsäure, verwendet werden; reduzierende trivalente anorganische oder organische Phosphorverbindungen, wie Phosphine, ferner Ester, Amide und Halogenide der phosphinigen, phosphonigen oder phosphorigen Säure, sowie diesen Phosphorsauerstoffverbindungen entsprechenden Phosphor-Schwefelverbindungen, worin organische Reste in erster Linie aliphatische, aromatische oder araliphatische Reste, z.B. gegebenenfalls substituierte Niederalkyl-, Phenyl oder Phenylniederalkylgruppen darstellen, wie z.B. Triphenylphosphin, Tri-n-butylphosphin, Diphenylphosphinigsäuremethylester, Diphenylchlorphosphin, Phenyldichlorphosphin, Benzolphosphonigsäuredimethylester, Butanphosphonigsäuremethylester, Phosphorigsäuretriphenylester, Phosphorigsäuretrimethylester, Phosphortrichlorid, Phosphortribromid, etc.; reduzierende Halogensilanverbindungen, die mindestens ein an das Siliciumatom gebundenes Wasserstoffatom aufweisen, und die ausser Halogen, wie Chlor, Brom oder Jod, auch organische Reste, wie aliphatische oder aromatische Gruppen, z.B. gegebenenfalls substituierte Niederalkyl- oder Phenylgruppen aufweisen können, wie Chlorsilan, Bromsilan, Di- oder Trichlorsilan, Di- oder Tribromsilan, Diphenylchlorsilan, Dimethylchlorsilan, Trimethyljodsilan oder auch Halogensilanverbindungen, worin alle Wasserstoffe durch organische Reste ersetzt sind, wie Triniederalkylhalogensilan, z.B. Trimethyljodsilan, oder cyclische, schwefelhaltige Silane, wie 1,3-Dithia-2,4-disilacyclobutane oder 1,3,5-Trithia-2,4,6-trisila-cyclohexane, deren Siliciumatome durch Kohlenwasserstoffreste, wie insbesondere Niederalkylreste substituiert sind, z.B. 2,2,4,4-Tetramethyl-1,3-dithia-2,4-disilacyclobutan oder 2,2,4,4,6,6-Hexamethyl-1,3,5-trithia-2,4,6-trisilacyclohexan, etc.; reduzierende quaternäre Chlormethylen-iminiumsalze, insbesondere -chloride oder -bromide, worin die Iminiumgruppe durch einen bivalenten oder zwei monovalente organische Reste, wie gegebenenfalls substituierte Niederalkylen- oder Niederalkylgruppen substituiert ist, wie N-Chlormethylen-N,N-diäthyliminiumchlorid oder N-Chlormethylen-pyrrolidiniumchlorid;und komplexe Metallyhdride, wie Natriumborhydrid, in Gegenwart von geeigneten Aktivierungsmitteln, wie Cobalt-II-chlorid, sowie Borandichlorid.

Als aktivierende Mittel, die zusammen mit denjenigen der obgenannten Reduktionsmittel verwendet werden, welche selber nicht Lewissäure-Eigenschaften aufweisen, d.h. die in erster Linie zusammen mit den Dithionit-, Jod- oder Eisen-II-cyanid- und den nicht-halogenhaltigen trivalenten Phosphor-Reduktionsmitteln oder bei der katalytischen Reduktion eingesetzt werden, sind insbesondere organische Carbon- und Sulfonsäurehalogenide, ferner Schwefel-, Phosphor- oder Siliciumhalogenide mit gleicher oder grösserer Hydrolysenkonstante zweiter Ordnung als Benzoylchlorid, z.B. Phosgen, Oxalylchlorid, Essigsäurechlorid oder -bromid, Chloressigsäurechlorid; Pivalinsäurechlorid, 4-Methoxybenzoesäurechlorid, 4-Cyanbenzoesäurechlorid, p-Toluolsulfonsäurechlorid, Methansulfonsäurechlorid, Thionylchlorid, Phosphoroxychlorid, Phosphortrichlorid, Phosphortribromid, Phenyldichlorphosphin, Benzolphosphonigsäuredichlorid, Dimethylchlorsilan oder Trichlorsilan, ferner geeignete Säureanhydride, wie Trifluoressigsäureanhydrid, oder cyclische Sultone, wie Aethansulton, 1,3-Propansulton, 1,4-Butansulton oder 1,3-Hexansulton zu erwähnen.

Die Reduktion wird vorzugsweise in Gegenwart von Lösungsmitteln oder Gemischen davon durchgeführt, deren Auswahl in erster Linie durch die Löslichkeit der Ausgangsstoffe und die Wahl des Reduktionsmittels bestimmt wird, so z.B. Niederalkancarbonsäuren oder Ester davon, wie Essigsäure und Essigsäureäthylester, bei der katalytischen Reduktion, und z.B. gegebenenfalls substituierte, wie halogenierte oder nitrierte aliphatische, cycloaliphatische, aromatische oder araliphatische Kohlenwasserstoffe, z.B. Benzol, Methylenchlorid, Chloroform oder Nitromethan, geeignete Säurederivate, wie Niederalkancarbonsäureester oder -nitrile, z.B. Essigsäureäthylester oder Acetonitril, oder Amide von anorganischen oder organischen Säuren, z.B. Dimethylformamid oder Hexamethylphosphoramid, Aether, z.B. Diäthyläther, Tetrahydrofuran oder Dioxan, Ketone, z.B. Aceton, oder Sulfone, insbesondere aliphatische Sulfone, z.B. Dimethylsulfon oder Tetramethylensulfon, etc., zusammen mit den chemischen Reduktionsmitteln, wobei diese Lösungsmittel vorzugsweise kein Wasser enthalten. Dabei arbeitet man gewöhnlicherweise bei Temperaturen von etwa -20° C bis etwa 100° C, wobei bei Verwendung von sehr reaktionsfähigen Aktivierungsmitteln die Reaktion bei tieferen Temperaturen durchgeführt werden kann.

Eine erhaltenen Verbindung der Formel I kann in eine andere Verbindung der Formel I umgewandelt werden, wobei die Reste $R_1$, $R_2$, $R_3$ oder A innerhalb des Rahmens ihrer Bedeutungen in andere Reste $R_1$, $R_2$, $R_3$ oder A übergeführt werden. Das betrifft in erster Linie die Abspaltung von Schutzgruppen, z.B. von $R_1$ und $R_2$ und von Schutzgruppen in den Resten $R_3$ und A, sowie die Ueberführung einer 4-Carboxylgruppe in eine physiologisch spaltbare veresterte 4-Carboxylgruppe.

In den erhaltenen Verbindungen der Formel I, worin funktionelle Gruppen geschützt sind, werden diese, z.B. geschützte Carboxyl-, Amino-, Hydroxy-, Mercapto- und/oder Sulfogruppen, in an sich bekannter Weise, wie mittels Solvolyse, insbesondere Hydrolyse, Alkoholyse oder Acidolyse, oder mittels Reduktion, insbesondere Hydrogenolyse oder chemische Reduktion, gegebenenfalls stufenweise oder gleichzeitig freigesetzt.

So kann man z.B. eine gegebenenfalls in 2-Stellung durch Silylgruppen substituierte Niederalkoxycarbonylgruppe, eine Polycycloalkoxycarbonyl- oder eine Diphenylmethoxycarbonylgruppe durch Behandeln mit einem geeigneten sauren Mittel, wie Ameisensäure oder Trifluoressigsäure, gegebenenfalls unter Zugabe einer nucleophilen Verbindung, wie Phenol oder Anisol, in die freie Carboxylgruppe überführen. Eine gegebenenfalls substituierte Benzyloxycarbonylgruppe kann z.B. mittels Hydrogenolyse durch Behandeln mit Wasserstoff in Gegenwart eines Hydrierkatalysators, wie eines Palladiumkatalysators freigesetzt werden. Ferner kann man bestimmt substituierte Benzyloxycarbonylgruppen, wie 4-Nitrobenzyloxycarbonyl, auch mittels chemischer Reduktion, z.B. durch Behandeln mit einem Alkalimetall-, z.B. Natriumdithionit, oder mit einem reduzierenden Metall, z.B. Zink, oder Metallsalz, wie einem Chrom-II-salz, z.B. Chrom-II-chlorid, üblicherweise in Gegenwart eines Wasserstoffabgebenden Mittels, das zusammen mit dem Metall nascierenden Wasserstoff zu erzeugen vermag, wie einer Säure, in erster Linie einer Carbonsäure oder α-Hydroxycarbonsäure, wie insbesondere Essigsäure, Ameisensäure, Glycolsäure, Diphenylglycolsäure, Milchsäure, Mandelsäure, p-Chlormandelsäure, Weinsäure, und dergleichen, oder eines Alkohols oder Thiols, wobei man vorzusweise Wasser zugibt, in die freie Carboxylgruppe überführen. Durch Behandeln mit einem reduzierenden Metall oder Metallsalz, wie oben beschrieben, kann man auch eine 2-Halogen-niederalkoxycarbonylgruppe (gegebenenfalls nach Umwandlung einer 2-Brom-niederalkoxycarbonylgruppe in eine 2-Iodniederalkoxycarbonylgruppe) oder eine Acylmethoxycarbonylgruppe in die freie Carboxylgruppe umwandeln, wobei eine Aroylmethoxycarbonylgruppe ebenfalls durch Behandeln mit einem nucleophilen, vorzugsweise salzbildenden Reagens, wie Natriumthiophenolat oder Natriumjodid gespalten werden kann. Eine substituierte Silyläthoxycarbonylgruppe kann auch durch Behandeln mit einem Salze der Fluorwasserstoffsäure, das Fluoridanion liefert, wie einem Alkalimetallfluorid, z.B. Natrium- oder Kaliumfluorid, in Anwesenheit eines macrocyclischen Polyäthers ("Kronenäther"), oder mit einem Fluorid einer organischen quaternären Base, wie Tetraalkylammoniumfluorid oder Trialkylarylammoniumfluorid, z.B. Tetraäthylammoniumfluorid oder Tetrabutylammoniumfluorid, in Gegenwart eines aprotischen polaren Lösungsmittels, wie Dimethylsulfoxid oder N,N-Dimethylacetamid, in die freie Carboxylgruppe übergeführt werden. Eine Polyhalogenaryloxycarbonylgruppe, wie die Pentachlorphenyloxycarbonylgruppe,wird unter milden basischen Bedingungen, wie durch verdünnte Natronlauge oder organische Basen in Gegenwart von Wasser, zur freien Carboxylgruppe verseift.

Eine z.B. durch Silylierung oder Stannylierung geschützte Carboxylgruppe kann in üblicher Weise, z.B. durch Behandeln mit Wasser oder einem Alkohol, freigesetzt werden.

Eine geschützte Aminogruppe wird in an sich bekannter und je nach Art der Schutzgruppe in verschiedenartiger Weise, z.B. mittels Solvolyse oder Reduktion, freigesetzt. Eine 2-Halogen-niederalkoxycarbonylaminogruppe (gegebenenfalls nach Umwandlung einer 2-Brom-niederalkoxycarbonylgruppe in eine 2-Jod-niederalkoxycarbonylgruppe), eine Acylmethoxycarbonylaminogruppe oder eine 4-Nitrobenzyloxycarbonylaminogruppe kann z.B. durch Behandeln mit einem geeigneten chemischen Reduktionsmittel, wie Zink in Gegenwart einer Carbon- oder α-Hydroxycarbonsäure, wie wässriger Essigsäure, eine Aroylmethoxycarbonylaminogruppe auch durch Behandeln mit einem nucleophilen, vorzugsweise salzbildenden Reagens, wie Natriumthiophenolat, und eine 4-Nitro-benzyloxycarbonylaminogruppe auch durch Behandeln mit einem Alkalimetall-, z.B. Natriumdithionit, eine Diphenylmethoxycarbonylamino-, tert.-Niederalkoxycarbonylamino-, 2-$(S_1)(S_2)(S_3)$-Silyläthoxycarbonylamino- oder Polycycloalkoxycarbonylaminogruppe durch Behandeln z.B. mit Ameisen- oder Trifluoressigsäure, eine gegebenenfalls substituierte Benzyloxycarbonylaminogruppe z.B. mittels Hydrogenolyse durch Behandeln mit Wasserstoff in Gegenwart eines Hydrierkatalysators, wie eines Palladiumkatalysators, eine Triarylmethylgruppe z.B. durch Behandelm mit wässriger Mineralsäure, und eine mit einer organischen Silyl- oder Stannylgruppe geschützte Aminogruppe z.B. mittels Hydrolyse oder Alkoholyse freigesetzt werden. Eine durch 2-Halogenacetyl, wie 2-Chloracetyl, geschützte Aminogruppe kann durch Behandeln mit Thioharnstoff in Gegenwart einer Base, oder mit einem Thiolatsalz, wie einem Alkalimetallthiolat des Thioharnstoffs und anschliessende Solvolyse, wie Alkoholyse oder Hydrolyse, des entstandenen Kondensationsproduktes freigesetzt werden. Eine durch eine substituierte-Silyläthoxycarbonylgruppe geschützte Aminogruppe kann auch durch Behandeln mit einem Salz der Fluorwasserstoffsäure, das Fluoridanionen liefert, wie oben bei der Freisetzung einer entsprechend geschützten Carboxylgruppe angegeben, in die freie Aminogruppe übergeführt werden. Eine Phosphor-, Phosphon- oder Phosphinamidogruppe kann durch Behandeln mit einer Phosphor-haltigen Säure, wie einer Phosphor-Phosphon- oder Phosphinsäure, z.B. Orthophosphorsäure oder Polyphosphorsäure, einem sauren Ester davon, z.B. Monomethyl-, Monoäthyl-, Dimethyl- oder Diäthylphosphat oder Monomethylphosphonsäure, oder einem Anhydrid davon, wie Phosphorpentoxid, und dergleichen, in die freie Aminogruppe übergeführt werden.

Eine durch eine Acylgruppe, eine Silyl- oder Stannylgruppe oder durch einen gegebenenfalls substituierten α-Phenylniederalkylrest geschützte Hydroxygruppe wird wie eine entsprechend geschützte Aminogruppe freigesetzt. Eine durch einen 2-oxa- oder 2-thia-aliphatischen oder -cycloaliphatischen Kohlenwasserstoffrest geschützte Hydroxygruppe wird durch Acidolyse freigesetzt.

Eine geschützte Sulfogruppe wird analog einer geschützten Carboxylgruppe freigesetzt.

Bevorzugt werden die Schutzgruppen so gewählt, dass sie alle gleichzeitig abgespalten werden können, beispielsweise acidolytisch, wie durch Behandeln mit Trifluoressigsäure oder Ameisensäure, oder reduktiv, wie durch Behandeln mit Zink und Eisessig, oder mit Wasserstoff und einem Hydrierkatalysator, wie einem Palladium/Kohle-Katalysator.

Die beschriebenen Spaltungsreaktionen werden unter an sich bekannten Bedingungen durchgeführt, wenn notwendig unter Kühlen oder Erwärmen, in einem geschlossenen Gefäss und/oder in einer Inertgas-, z.B. Stickstoffatmosphäre.

Die Ueberführung einer freien Carboxylgruppe in einer erhaltenen Verbindung der Formel (I) in eine veresterte Carboxylgruppe, die unter physiologischen Bedingungen spaltbar ist, erfolgt nach an sich bekannten Veresterungsmethoden, beispielsweise indem man eine Verbindung der Formel(I), worin andere funktionelle Gruppen, wie Amino-, Hydroxy- oder Sulfogruppen, gegebenenfalls in geschützter Form vorliegen, oder ein bezüglich der zu veresternden Carboxylgruppe reaktionsfähiges funktionelles Derivat davon, oder ein Salz davon, mit einem entsprechenden Alkohol oder einem reaktionsfähigen funktionellen Derivat davon, verestert.

In einer erhaltenen Verbindung der Formel (I), kann eine Hydroxymethylengruppe A zu einer Oxomethylengruppe oxidiert werden. Die Oxidation kann, gegebenenfalls unter Schutz einer freien Amino- und Carboxylgruppe, auf an sich bekannte, d.h. wie für die Oxidation von Hydroxy- zu Oxogruppen bekannte Weise durchgeführt werden. Als Oxidationsmittel kommen oxidierende Oxide, wie solche des Mangans, Chroms, Stickstoffs oder Schwefels, wie Mangandioxid, Chromtrioxid, z.B. Jones Reagens oder Chromtrioxid in Gegenwart von Essigsäure, Schwefelsäure oder Pyridin, Distickstofftetroxid, Dimethylsulfoxid gegebenenfalls in Gegenwart von Dicyclohexylcarbodiimid oder Sauerstoff, und Peroxide, wie Wasserstoffperoxid, sauerstoffhaltige Säuren, wie Permangansäure, Chromsäure oder Hypochlorsäure oder Salze davon, wie Kaliumpermanganat, Natrium- oder Kaliumdichromat oder Kaliumhypochlorit, in Frage. Die Hydroxymethylengruppe kann auch durch die Oppenauer-Oxidation in die Oxomethylengruppe übergeführt werden, d.h. durch Behandeln mit dem Salz eines sterisch gehinderten Alkohols, wie Aluminium- oder Kalium-tert.-butoxid, -isopropoxid oder -phenoxid in Gegenwart eines Ketons, wie Aceton, Cyclohexanon oder Fluorenon. Eine weitere Möglichkeit die Hydroxymethylengruppe in die Oxomethylengruppe überzuführen besteht in der Dehydrierung, z.B. mit Raney-Nickel.

Die Oxidation wird je nach Oxidationsmittel in Wasser oder einem gegebenenfalls wasserhaltigen Lösungsmittel bei Temperaturen von etwa 0° bis etwa 100° durchgeführt.

In einer erhaltenen Verbindung der Formel (I), worin A eine Oxomethylengruppe bedeutet, kann diese, gegebenenfalls unter Schutz funktioneller Gruppen, durch

Behandeln mit Hydroxylamin oder O-Methylhydroxylamin in eine Hydroxyiminomethylen- bzw. Methoxyiminomethylengruppe übergeführt werden.

Die Reaktion der Oxomethylengruppe mit der Hydroxylaminverbindung wird auf übliche Weise ausgeführt, z.B. indem man die beiden Reaktionspartner in einem Lösungsmittel, wie Wasser oder einem organischen Lösungsmittel, wie einem Alkohol, z.B. Methanol, bei leicht erhöhter oder erniedrigter Temperatur, gegebenenfalls in einer Inertgas-, wie Stickstoffatmosphäre, reagieren lässt. Die Hydroxylaminverbindung kann, auch in situ, aus einem ihrer Salze, beispielsweise einem Hydrohalogenid, wie Hydrochlorid, durch Behandeln mit einer anorganischen Base, wie einem Alkalimetallhydroxid, z.B. Natriumhydroxid, oder einer organischen Base, wie einem tertiären Amin, z.B. einem Triniederalkylamin, wie Triniederalkylamin, wie Triäthylamin oder Aethyl-diisopropylamin, oder einer heterocyclischen tertiären Base, wie Pyridin, in Freiheit gesetzt werden.

In einer erhaltenen Verbindung der Formel (I), worin A Methylen bedeutet, kann die Methylengruppe durch Nitrosierung, z.B. durch Behandeln mit einem Nitrit, wie Amylnitrit, in Gegenwart einer Base, z.B. anlaog BE 855 953, in eine Hydroxyiminogruppe übergeführt werden, die anschliessend, falls erwünscht, durch Methylierung, z.B. mit Dimethylsulfat in Gegenwart einer Base, in eine Methoxyiminomethylengruppe übergeführt werden kann. Ferner kann eine Methylengruppe A zu einer Sulfomethylengruppe sulfonyliert werden, z.B. durch Behandeln mit dem $SO_3$/ Dioxan Komplex, z.B. analog der DT-OS 2 638 028.

Salze von Verbindungen der Formel (I) können in an sich bekannter Weise hergestellt werden. So kann man Salze von Verbindungen der Formel I mit sauren Gruppen,

z.B. durch Behandeln mit Metallverbindungen, wie Alkalimetallsalzen von geeigenten Carbonsäuren, z.B. dem Natriumsalz der α-Aethylcapronsäure oder Natriumbicarbonat, oder mit Ammoniak oder einem geeigneten organischen Amin bilden, wobei man vorzugsweise stöchiometrische Mengen oder nur einen kleinen Ueberschuss des salzbildenden Mittels verwendet. Säureadditionssalze von Verbindungen der Formel I erhält man in üblicher Weise, z.B. durch Behandeln mit einer Säure oder einem geeigneten Anionenaustauschreagens. Innere Salze von Verbindungen der Formel(I),welche eine freie Carboxylgruppe enthalten, können z.B. durch Neutralisieren von Salzen, wie Säureadditionssalzen, auf den isoelektrischen Punkt, z.B. mit schwachen Basen, oder durch Behandeln mit flüssigen Ionenaustauschern gebildet werden.

Die Verfahren umfassen auch diejenigen Ausführungsformen, wonach als Zwischenprodukte anfallende Verbindungen als Ausgangsstoffe verwendet und die restlichen Verfahrensschritte mit diesen durchgeführt werden, oder das Verfahren auf irgendeiner Stufe abgebrochen wird; ferner können Ausgangsstoffe in Form von Derivaten verwendet oder in situ, gegebenenfalls unter den Reaktionsbindungen, gebildet werden.

Die Ausgangsverbindungen der Formeln II bis IV sind bekannt oder können analog bekannten Verfahren hergestellt werden.

So sind Ausgangsverbindungen der Formel II z.B. aus der DT-OS 2 537 974 und der Formel III z.B. aus BE 852 971, BE 853 545, DT-OS 2 556 736 und DT-OS 2 638 028 bekannt.

Ausgangsverbindungen der Formel IV können durch Acylierung von Verbindungen der Formel II mit einer Säure der Formel $X\text{-}CH_2\text{-}CO\text{-}A\text{-}COOH$ oder einem reaktionsfä-

higen funktionellem Derivat davon hergestellt werden.

Ausgangsverbindungen der Formel V sind bisher nicht bekannt geworden. Sie können, analog der oben beschriebenen Acylierung von Verbindungen der Formel II, z.B. durch Acylierung von bekannten oder auf an sich bekannte Weise herstellbaren 7β-Amino-3-Y-3-cephem-4-carbonsäureverbindungen (VII), worin die 4-Carboxylgruppe durch den Rest $R_2$ geschützt sein kann, hergestellt werden. In einer erhaltenen Verbindung der Formel V kann eine Gruppe Y auf an sich bekannte Weise in eine andere Gruppe Y übergeführt werden.

Ausgangsverbindungen der Formel VI sind neu. Sie können gemäss den Verfahren a) bis c) erhalten werden, sofern unter basischen Bedingungen gearbeitet wird.

Die neuen Ausgangstoffe und Verfahren zu ihrer Herstellung sind ebenfalls Gegenstand der vorliegenden Erfindung.

Die pharmakologisch verwendbaren Verbindungen der vorliegenden Erfindung können z.B. zur Herstellung von pharmazeutischen Präparaten verwendet werden, welche eine wirksame Menge der Aktivsubstanz zusammen oder im Gemisch mit anorganischen oder organischen, festen oder flüssigen, pharmazeutisch verwendbaren Trägerstoffen enthalten, die sich zur enteralen oder parenteralen Verabreichung eignen. Zur enteralen Verabreichung verwendet man Tabletten oder Gelatinekapseln, welche den Wirkstoff zusammen mit Verdünnungsmitteln, z.B. Laktose, Dextrose, Sukrose, Mannitol, Sorbitol, Cellulose und/oder Glycin, und Schmiermitteln, z.B. Kieselerde, Talk, Stearinsäure oder Salze davon, wie Magnesium- oder Calciumstearat, und/oder Polyäthylenglykol, aufweisen; Tabletten enthalten ebenfalls Bindemittel, z.B. Magnesiumaluminiumsilikat, Stärken, wie Mais-, Weizen-, Reis- oder Pfeilwurzstärke, Gelatine, Traganth, Methylcellulose, Natriumcarboxymethylcellulose und/oder Polyvinylpyrrolidon, und, wenn erwünscht, Sprengmittel, z.B. Stärken, Agar, Alginsäure oder ein Salz davon, wie Natriumalginat, und/oder Brausemischungen, oder Adsorptionsmittel, Farbstoffe, Geschmackstoffe und Süssmittel. Suppositorien sind in erster Linie Fettemulsionen oder -suspensionen.

Vorzugsweise verwendet man die pharmakologisch wirksamen Verbindungen der vorliegenden Erfindung in Form von injizierbaren, z.B. intravenös oder subcutan, verabreichbaren Präparaten oder von Infusionslösungen. Solche Lösungen sind vorzugsweise isotonische wässrige Lösungen oder Suspensionen, wobei diese z.B. aus lyophilisierten Präparaten, welche die Wirksubstanz allein oder zusammen mit einem Trägermaterial, z.B. Mannit, enthalten, vor Gebrauch hergestellt werden können. Die pharmazeutischen Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Druckes und/oder Puffer enthalten. Die vorliegenden pharmazeutischen Präparate, die, wenn erwünscht, weitere pharmakologisch wertvolle Stoffe enthalten können, werden in an sich bekannter Weise, z.B. mittels konventioneller Mischungs-, Lösungs-, oder Lyophilisierungsverfahren, hergestellt und enthalten von etwa 0,1% bis 100%, insbesondere von etwa 1% bis etwa 50%, Lyophilisate bis zu 100% des Aktivstoffes. Je nach Art der Infektion, Zustand des infizierten Organismus verwendet man tägliche Dosen von etwa 0,5 g bis etwa 5 g s.c. zur Behandlung von Warmblütern von etwa 70 kg Gewicht.

Die folgenden Beispiele dienen zur Illustration der Erfindung; Temperaturen werden in Celsiusgraden angegeben.

Rf-Angaben für Dünnschichtchromatographie:

DC: Auf Silicagel-Fertigplatten SL 254 der Fa. Antec, Birsfelden; System 96: Butanol:Essigsäure:Wasser 67:10:23.

Bei den Methoxyiminoessigsäurederivaten handelt es sich immer um das Z-Isomere.

Beispiel 1:

a) Eine Lösung von 313 mg (0,9 mMol) 2-[2-(2-Trimethylsilyläthoxycarbonylamino)-4-thiazolyl]-2-methoxyiminoessigsäure in 3 ml Methylenchlorid und 0,085 ml (1,05 mMol) Pyridin wird bei -15° unter Stickstoff mit 0,165 ml (1,1 mMol) Diäthylphosphorobromidat versetzt und 20 Minuten bei dieser Temperatur gerührt. Nach Zugabe von 0,58 g (0,9 mMol) 7β-Amino-3-(1-methyltetrazol-5-ylthio)-3-cephem-4-carbonsäure-diphenylmethylester (~ 74%-ig) bei -15° wird die Reaktionslösung bei Raumtemperatur während 4 Stunden gerührt, dann mit Chloroform verdünnt, nacheinander mit verdünnter Schwefelsäure, Wasser, wässriger Natriumbicarbonatlösung und wässriger Natriumchloridlösung gewaschen, über Natriumsulfat getrocknet und im Vakuum eingedampft. Der Rückstand wird durch präparative Dickschichtchromatographie an Silicagel mit Toluol/Aethylacetat 1:1 gereinigt. Man erhält den 7β-{2-[2-(2-Trimethylsilyläthoxycarbonylamino)-4-thiazolyl]-2-methoxyiminoacetamido}-3-(1-methyltetrazol-5-ylthio)-3-cephem-4-carbonsäurediphenylmethylester vom Rf-Wert 0,54 (Silicagel; Toluol/Aethylacetat 1:1) als Schaum. IR-Spektrum (Methylenchlorid): charakteristische Banden bei 2,9; 3,15; 5,57; 5,76; 5,92; 6,44 μ.

Als Nebenprodukt wird der 7β-{2-[2-(2-Trimethylsilyläthoxycarbonylamino)-4-thiazolyl]-2-methoxyiminoacetamido}-2-(1-methyltetrazol-5-ylthio)-2-cephem-4-carbonsäurediphenylmethylester vom Rf-Wert 0,39 (Silicagel, Toluol/Aethylacetat 1:1) isoliert. IR-Spektrum (Methylenchlorid): charakteristische Banden bei 2,90; 3,19; 5,58; 5,77; 5,92; 6,43 μ.

Die Ausgangsmaterialien können wie folgt erhalten werden:

b) Eine Suspension von 2,98 g (14,3 mMol) Phosphorpentachlorid in 60 ml Methylenchlorid wird bei 0° und unter Stickstoff mit 1,27 ml (16,1 mMol) Pyridin versetzt. Nach 10 Minuten fügt man 7,5 g (12,2 mMol) 7β-Phenoxyacetylamino-3-(1-methyltetrazol-5-ylthio)-3-cephem-4-carbonsäure-diphenylmethylester zu und rührt während 2 1/2 Stunden bei 0°. Nach Zugabe von 7,7 ml Isobutanol bei -20° wird die Reaktionsmischung während 1 Stunde bei 0° stehen gelassen. Die Lösung wird mit 19 ml Wasser versetzt und während 10 Minuten bei Raumtemperatur gut gerührt. Die organische Phase wird abgetrennt, mit verdünnter Bicarbonatlösung und wässriger Natriumchloridlösung gewaschen, über Natriumsulfat getrocknet und im Vakuum eingedampft. Der Rückstand wird mit Aether versetzt und der ausfallende Festkörper mit viel Aether gewaschen. Man erhält so den 7β-Amino-3-(1-methyltetrazol-5-yl-thio)-3-cephem-4-carbonsäure-diphenylmethylester vom Rf-Wert 0,29 (Toluol/Essigester 1:3). IR-Spektrum (Methylenchlorid): 3,3; 5,59; 5,86 μ.

c) Eine Lösung von 10 g (465 mMol) 2-(2-Aminothiazol-4-yl)-2-methoxyimino-essigsäuremethylester in 50 ml Methylenchlorid und 6 ml (74 mMol) Pyridin wird bei 0° und unter Stickstoff mit 14 ml (74 mMol) Chlorameisensäure-trimethylsilyläthylester versetzt und 3 Stunden bei Raumtemperatur gerührt. Die Reaktionslösung wird nacheinander mit verdünnter Schwefelsäure, Wasser, wässriger Natriumbicarbonatlösung und wässriger Natriumchloridlösung gewaschen, über Natriumsulfat getrocknet und im Vakuum eingedampft. Der Rückstand wird an 300 g Silicagel mit dem Laufmittelsystem Toluol/Aethylacetat 9:1 chromatographiert. Man erhält den 2-[2-(2-Trimethylsilyläthoxycarbonylamino)-4-thiazolyl]-2-methoxyiminoessigsäuremethylester vom Rf =

0,36 (Silicagel; Toluol/Aethylacetat 9:1). IR-Spektrum (Methylenchlorid): charakteristische Banden bei 2,94; 5,72; 6,41 µ.

d) Eine Lösung von 9,1 g (25,3 mMol) 2-[2-(2-Trimethylsilyläthoxycarbonylamino)-4-thiazolyl]-2-methoxyiminoessigsäuremethylester in 100 ml Aethanol wird mit einer Lösung von 9 g (160 mMol) Kaliumhydroxid in 80 ml Wasser und 100 ml Aethanol versetzt und während 2 Stunden bei Raumtemperatur gerührt. Anschliessend wird mit 35 ml Salzsäure (4 N) neutral gestellt und das Aethanol am Rotationsverdampfer abgezogen. Die wässrige Lösung wird mit Aethylacetat überschichtet und der pH mit verdünnter Salzsäure auf 2 eingestellt. Die wässrigen Phasen werden zweimal mit Aethylacetat extrahiert und die organischen Lösungen mit wässriger Natriumchloridlösung gewaschen, über Natriumsulfat getrocknet und im Vakuum eingedampft. Der Rückstand wird aus Aethylacetat/Aether kristallisiert. Man erhält die 2-[2-(2-Trimethylsilyläthoxycarbonylamino)-4-thiazolyl]-2-methoxyiminoessigsäure vom Rf-Wert 0,5 (Silicagel; System 96) (Smp. 173°; IR-Spektrum (Nujol): charakteristische Banden bei 3,12; 5,77; 5,84; 6,35 µ.

<u>Beispiel 2:</u> Eine Lösung von 786 mg (0,97 mMol) 7β-{2-[2-(2-Trimethylsilyläthoxycarbonylamino)-4-thiazolyl]-2-methoxyiminoacetamido}-3-(1-methyltetrazol-5-yl-thio)-2-cephem-4-carbonsäure-diphenylmethylester in 7 ml Chloroform wird auf 0° gekühlt, unter guter Kühlung mit 205 mg (1,05 mMol) m-Chlorperbenzoesäure (90 %-ig) versetzt und 1/4 Stunde gerührt. Hierauf wird überschüssige Persäure durch Zugabe von Natriumthiosulfat zerstört. Die Reaktionslösung wird

mit wässriger Natriumbicarbonat und gesättigter wässriger Kochsalzlösung extrahiert, über Natriumsulfat getrocknet und eingeengt. Der Rückstand wird durch präparative Dickschichtchromatographie (Silicagel, Toluol/Aethylacetat 1:2) gereinigt und ergibt das 7β-{2-[2-(2-Trimethylsilyläthoxycarbonylamino)-4-thiazolyl]-2-methoxyiminoacetamido}-3-(1-methyltetrazol-5-ylthio)-3-cephem-4-carbonsäurediphenylmethylester-1β-oxid, DC (Silicagel): Rf = 0,28 (Toluol/Aethylacetat 1:2). IR-Spektrum (Methylenchlorid): charakteristische Banden bei 2,94; 3,12; 5,51; 5,76; 5,89; 6,42 μ.

<u>Beispiel 3</u>: Eine Lösung von 332 mg (0,41 mMol) 7β-{2-[2-(2-Trimethylsilyläthoxycarbonylamino)-4-thiazolyl]-2-methoxyiminoacetamido}-3-(1-methyltetrazol-5-yl-thio)-3-cephem-4-carbonsäure-diphenylmethylester-1β-oxid in 5 ml Methylenchlorid und 0,3 ml DMF wird auf 0° gekühlt, mit 0,155 ml (1,6 mMol) Phosphortrichlorid (destilliert) versetzt und 30 Minuten gerührt. Die Reaktionslösung wird mit wässriger Natriumbicarbonatlösung und gesättigter wässriger Kochsalzlösung extrahiert, die organische Phase über Natriumsulfat getrocknet und am Rotationsverdampfer eingeengt. Der Rückstand wird durch präparative Dickschichtchromatographie an Silicagel mit Toluol/Aethylacetat 1:1 gereinigt. Man erhält den 7β-{2-[2-(2-Trimethylsilyläthoxycarbonylamino)-4-thiazolyl]-2-methoxyiminoacetamido}-3-(1-methyltetrazol-5-ylthio)-3-cephem-4-carbonsäure-diphenylmethylester vom Rf-Wert 0,54 (Silicagel; Toluol/Aethylacetat 1:1) als Schaum.

<u>Beispiel 4</u>: Eine Mischung von 1,4 g (17,3 mMol) 7β-{2-[2-(2-Trimethylsilyläthoxycarbonylamino)-4-thiazolyl]-2-methoxyiminoacetamido}-3-(1-methyltetrazol-5-ylthio)-3-cephem-4-carbonsäure-diphenylmethylester und 3 ml Anisol wird bei Raumtemperatur mit 12 ml Trifluoressigsäure versetzt und während 15 Minuten gerührt, dann mit Toluol verdünnt und anschliessend am Rotationsverdampfer eingeengt (zweimal wiederholt). Der Rückstand wird in Wasser aufgenommen und mit verdünnter Natronlauge auf pH 7 eingestellt. Die Substanz wird an 500 ml Amberlite XAD 2 aufgezogen. Der Säuleninhalt wird mit 1,5 l Wasser gewaschen, danach kann das Natriumsalz der 7β-[2-(2-Amino-4-thiazolyl)-2-methoxyiminoacetamido]-3-(1-methyltetrazol-5-ylthio)-3-cephem-4-carbonsäure mit dem Gemisch Wasser/-15% Isopropanol ausgewaschen werden. Nach Lyophilisation der entsprechenden Fraktionen fällt das Produkt mit dem Rf-Wert 0,44 (Silicagel; System 96) als beiges Pulver an.

Beispiel 5: Eine Lösung von 0,2 g (0,25 mMol) 7β-{2-[2-(2-Trimethylsilyläthoxycarbonylamino)-4-thiazolyl]-2-methoxyiminoacetamido}-3-methansulfonyloxy-3-cephem-4-carbonsäure-diphenylmethylester in 1,5 ml Tetrahydrofuran wird bei Raumtemperatur unter Stickstoff mit 0,105 g (0,76 mMol) Natrium-1-methyltetrazol-5-thiolat versetzt und bei gleicher Temperatur während 2 Stunden gerührt. Das Lösungsmittel wird im Vakuum abgedampft, der Rückstand in Methylenchlorid aufgenommen und diese Lösung zweimal mit wässriger Natriumchloridlösung gewaschen, über Natriumsulfat getrocknet und im Vakuum eingedampft. Man erhält den 7β-{2-[2-(2-Trimethylsilyläthoxycarbonylamino)-4-thiazolyl]-2-methoxyiminoacetamido}-3-(1-methyltetrazol-5-ylthio)-3-cephem-4-carbonsäure-diphenylmethylester vom Rf-Wert 0,54 (Silicagel; Toluol/Aethylacetat 1:1) als Schaum.

Das Ausgangsmaterial kann wie folgt hergestellt werden:

b) Eine Suspension von 4,2 g (20,0 mMol) Phosphorpentachlorid in 84 ml Methylenchlorid wird bei 0° und unter Stickstoff mit 1,79 ml Pyridin versetzt. Nach 10 Minuten fügt man 10,23 g 7β-Phenoxyacetylamino-3-methansulfonyloxy-3-cephem-4-carbonsäure-diphenylmethylester zu und rührt während 2 1/2 Stunden bei 0°. Nach Zugabe von 10,8 ml Isobutanol bei -15° wird bei 0° während einer Stunde stehen gelassen. Die Lösung wird mit 27 ml Wasser versetzt und während 10 Minuten bei Raumtemperatur gut gerührt. Die organische Phase wird abgetrennt, mit verdünnter Bicarbonatlösung und wässriger Natriumchloridlösung gewaschen, über Natriumsulfat getrocknet und im Vakuum eingedampft. Der Rückstand wird mit Aether versetzt und der ausgefallene Festkörper mit viel Aether gewaschen. Man erhält so den 7β-Amino-3-methansulfonyloxy-3-cephem-4-carbonsäurediphenylmethylester vom Rf-Wert 0,38 (Silicagel; Toluol/Aethylacetat 1:1). IR-Spektrum (Methylenchlorid): 2900; 1790; 1725 $cm^{-1}$.

c) Eine Lösung von 313 mg (0,9 mMol) 2-[2-(2-Trimethylsilyläthoxycarbonylamino)-4-thiazolyl]-2-methoxyiminoessigsäure in 3 ml Methylenchlorid und 0,085 ml (1,05 mMol) Pyridin wird bei -15° unter Stickstoff mit 0,33 ml (2,2 mMol) Diäthylphosphorobromidat versetzt und 20 Minuten gerührt. Nach Zugabe von 414 mg (0,9 mMol) 7β-Amino-3-methansulfonyloxy-3-cephem-4-carbonsäurediphenylmethylester bei -15° wird die Reaktionslösung bei Raumtemperatur während 2 Stunden gerührt, dann mit Chloroform verdünnt, nacheinander mit verdünnter Schwefelsäure, Wasser, wässriger Natriumbicarbonatlösung und wässriger Natriumchloridlösung gewaschen, über Natriumsulfat getrocknet und im Vakuum eingedampft.

Der Rückstand wird durch präparative Dickschichtchromatographie an Silicagel mit Toluol/Aethylacetat 1:1 gereinigt. Man erhält den 7β-{2-[2-(2-Trimethylsilyläthoxycarbonylamino)-4-thiazolyl]-2-methoxyiminoacetamido}-3-methansulfonyloxy-3-cephem-4-carbonsäure-diphenylmethylester vom Rf-Wert 0,5 (Silicagel; Toluol/Aethylacetat 1:1) als Schaum. IR-Spektrum (Methylenchlorid): charakteristische Banden bei 2,93; 3,06; 5,56; 5,75; 5,90; 6,4μ. Als Nebenprodukt wird wenig vom 7β-{2-[2-(2-Trimethylsilyläthoxycarbonylamino)-4-thiazolyl]-2-methoxyiminoacetamido}-3-methansulfonyloxy-2-cephem-4-carbonsäure-diphenylmethylester vom Rf-Wert 0,37 (Silicagel; Toluol/Aethylacetat 1:1) isoliert. IR-Spektrum (Methylenchlorid): Banden bei 2,93; 3,06; 5,58; 5,70; 5,90; 6,4 μ.

Beispiel 6:

a) Eine Lösung von 840 mg (2,75 mMol) 2-(2-tert.Butoxycarbonylamino-4-thiazolyl)-2-methoxyiminoessigsäure in 9 ml Methylenchlorid und 0,26 ml (3,23 mMol) Pyridin wird bei -15° unter Stickstoff mit 0,5 ml (3,4 mMol) Diäthylphosphorobromidat versetzt und 30 Minuten bei dieser Temperatur gerührt. Nach Zugabe von 1,82g(2,75 mMol) 7β-Amino-3-(1-methyltetrazol-5-ylthio)-3-cephem-4-carbonsäure-diphenylmethylester (~ 74%-ig) bei -15° wird die Reaktionslösung bei Raumtemperatur während 2 Stunden gerührt, dann mit Chloroform verdünnt, nacheinander mit verdünnter Schwefelsäure, Wasser, wässriger Natriumbicarbonatlösung und wässriger Natriumchloridlösung gewaschen, über Natriumsulfat getrocknet und im Vakuum eingedampft. Der Rückstand wird durch präparative Dickschichtchromatographie an Silicagel mit Toluol/Aethylacetat 1:1 gereinigt. Man erhält den 7β-[2-(2-tert.Butoxycarbonylamino-4-thiazolyl)-2-methoxyiminoacetamido]-3-(1-methyltetrazol-5-ylthio)-3-cephem-4-carbonsäurediphenylmethylester vom Rf-Wert 0,32 (Silicagel; Toluol/Aethylacetat 1:1) als Schaum. IR-Spektrum (Methylenchlorid): charakteristische Banden bei 2,95; 3,15; 5,58; 5,80; 5,91; 6,47 μ.

Das Ausgangsmaterial kann wie folgt erhalten werden:

b) Zu einer Lösung von 30,6 ml (60 mMol) Phosgen in Toluol (~ 20 %-ig) und 80 ml Tetrahydrofuran wird bei -20° und unter Stickstoff ein Gemisch von 10,8 g (50 mMol) 2-(2-Amino-4-thiazolyl)-2-methoxyimino-essigsäuremethylester, 8,4 ml (60 mMol) Triäthylamin und 100 ml Tetrahydrofuran innerhalb von 10 Minuten zugetropft. Anschliessend wird während 5 Minuten bei -20° nachgerührt. Zur Reaktionslösung werden bei -20° unter gutem Kühlen und unter Stickstoff 21 ml (150 mMol) Triäthylamin in 100 ml tert.Butanol zugetropft (Zutropfdauer 15 Minuten). Dann wird das Kühlbad entfernt und die Reaktionslösung bei Raumtemperatur während 1 3/4 Stunden gerührt. Die Reaktionslösung wird mit Aethylacetat verdünnt und nacheinander mit verdünnter Schwefelsäure, Wasser, wässriger Natriumbicarbonatlösung und wässriger gesättigter Natriumchloridlösung gewaschen, über Natriumsulfat getrocknet und im Vakuum eingedampft. Der Rückstand wird an 440 g Silicagel mit dem Laufmittelsystem Toluol/Aethylacetat 6:1 chromatographiert und der 2-(2-tert.Butoxycarbonylamino-4-thiazolyl)-2-methoxyimino-essigsäuremethylester mit dem Rf-Wert 0,39 (Silicagel; Toluol/Aethylacetat 6:1) als Schaum isoliert. IR-Spektrum (Methylenchlorid): charakteristische Banden bei 2,94; 5,74; 5,79; 6,47 μ.

c) Eine Lösung von 19,0 g (60,3 mMol) 2-(2-tert. Butoxycarbonylamino-4-thiazolyl)-2-methoxyiminoessigsäuremethylester in 240 ml Aethanol wird mit einer Lösung von 21,4 g (381 mMol) Kaliumhydroxid (86 %-ig) in 190 ml Wasser und 240 ml Aethanol versetzt und während 2 Stunden bei Raumtemperatur gerührt. Anschliessend wird mit 50 ml Salzsäure (4N) neutral gestellt und das Aethanol am Rotationsverdampfer abgezogen. Die wässrige Lösung wird mit Aethylacetat überschichtet und der pH mit verdünnter Salzsäure auf 2 eingestellt. Die wässrigen Phasen werden zweimal mit Aethylacetat extrahiert und die organischen Lösungen mit wässriger Natriumchloridlösung gewaschen, über Natriumsulfat getrocknet und im Vakuum eingedampft. Der Rückstand wird aus Aethylacetat/Aether/Hexan kristallisiert. Man erhält die 2-(2-tert.Butoxycarbonylamino)-2-methoxyiminoessigsäure vom Rf-Wert 0,43 (Silicagel; System 96); Smp. 167° (Zersetzung); IR-Spektrum (Nujol): charakteristische Banden bei 3,11; 5,79; 6,35 µ.

<u>Beispiel 7:</u> Eine Mischung von 1,23 g (1,6 mMol) 7β-[2-(2-tert.Butoxycarbonylamino-4-thiazolyl)-2-methoxyiminoacetamido]-3-(1-methyltetrazol-5-ylthio)-3-cephem-4-carbonsäure-diphenylmethylester und 3 ml Anisol wird bei Raumtemperatur mit 12 ml Trifluoressigsäure versetzt und während 15 Minuten gerührt, dann mit Toluol verdünnt und anschliessend am Rotationsverdampfer eingeengt (zweimal wiederholt). Der Rückstand wird mit Aether verrieben und das unlösliche Pulver durch Filtration isoliert. Dieses wird in Wasser gelöst und die wässrige Phase auf pH 7 gestellt. Das Natriumsalz der 7β-[2-(2-Amino-4-thiazolyl)-

2-methoxyiminoacetamido]-3-(1-methyltetrazol-5-ylthio)-3-cephem-4-carbonsäure wird durch präparative Schichtchromatographie auf DC-Trägerplatten "UP $C_{12}$" (Firma Antec) mit dem Laufmittelsystem Acetonitril/Wasser 1:4 isoliert. Rf-Wert 0,56 ("UP $C_{12}$"; Acetonitril/$H_2O$ 1:3).

Beispiel 8: Eine Lösung von 1,3 g (1,65 mMol) 7β-{2-[2-(2-Trimethylsilyläthoxycarbonylamino)-4-thiazolyl]-2-methoxyiminoacetamido}-3-methansulfonyloxy-3-cephem-4-carbonsäure-diphenylmethylester in 9 ml Tetrahydrofuran wird bei Raumtemperatur unter Stickstoff mit 0,8 g (4,6 mMol) 1-(2-Dimethylaminoäthyl)-tetrazol-5-ylthiol und 0,27 ml (1,8 mMol) 1,5-Diazabicyclo[5.4.0]undec-5-en versetzt und bei gleicher Temperatur während 2½ Stunden gerührt. Das Lösungsmittel wird im Vakuum abgedampft, der Rückstand in Methylenchlorid aufgenommen und diese Lösung zweimal mit wässriger Natriumchloridlösung gewaschen, über Natriumsulfat getrocknet und im Vakuum eingedampft. Man erhält ein Gemisch von 7β-{2-[2-(2-Trimethylsilyläthoxycarbonylamino)-4-thiazolyl]-2-methoxyiminoacetamido}-3-[1-(2-dimethylaminoäthyl)-tetrazol-5-ylthio]-3-cephem-4-carbonsäure-diphenylmethylester und dem entsprechenden 2-Cephem-4-carbonsäureester vom Rf-Wert 0,32 (Silicagel: Toluol/Aethylacetat 1:3) als Schaum. IR-Spektrum (Methylenchlorid): 2,94; 3,11; 3,54; 3,61; 5,61; 5,91; 6,47 μ.

Beispiel 9: 479 mg (0,55 mMol) eines Gemisches von 7β-{2-[2-(2-Trimethylsilyläthoxycarbonylamino)-4-thiazolyl]-2-methoxyiminoacetamido}-3-[1-(2-dimethylaminoäthyl)-tetrazol-5-ylthio]-3-cephem-4-carbonsäure-diphenylmethylester und 7β-{2-[2-(2-Trimethylsilyläthoxycarbonylamino)-4-thiazolyl]-2-methoxyiminoacetamido}-3-[1-(2-dimethylaminoäthyl)-tetrazol-5-ylthio)-2-cephem-4-carbonsäurediphenylmethylester (aus Beispiel 8) werden in 9 ml Chloroform gelöst und auf 0° gekühlt, mit 0,084 ml (1,1 mMol) m-Chlorperbenzoesäure (90%-ig) versetzt und 3/4 Stunden bei 0° gerührt.Hierauf wird überschüssige Persäure durch Zugabe von Natriumthiosulfat zerstört. Die Reaktionslösung wird mit wässriger Natriumbicarbonatlösung und gesättigter wässriger Kochsalzlösung extrahiert, über Natriumsulfat getrocknet und eingeengt. Der Rückstand wird durch präparative Dickschichtchromatographie (Silicagel, Toluol/Aethylacetat 1:3) gereinigt und ergibt das 7β-{2-[2-(2-Trimethylsilyläthoxycarbonylamino)-4-thiazolyl]-2-methoxyiminoacetamido}-3-[1-(2-dimethylaminoäthyl)-tetrazol-5-ylthio)-3-cephem-4-carbonsäurediphenylmethylester-1β-oxid, DC (Silicagel): Rf = 0,27 (Toluol/Aethylacetat 1:3). IR-Spektrum (Methylenchlorid): charakteristische Banden bei 2,96; 3,12; 5,52; 5,79; 5,91; 6,45 μ.

<u>Beispiel 10:</u> Eine Lösung von 200 mg (0,22 mMol) 7β-{2-[2-(2-Trimethylsilyl-äthoxycarbonylamino)-4-thiazolyl]-2-methoxyiminoacetamido}-3-[1-(2-dimethylaminoäthyl)-tetrazol-5-ylthio)-3-cephem-4-carbonsäurediphenylmethylester-1β-oxid in 2,5 ml Methylenchlorid und 0,7 ml Dimethylformamid wird auf 0° gekühlt, mit 0,078 ml (0,8 mMol) Phosphortrichlorid (destilliert) versetzt und bei Raumtemperatur 10 Minuten gerührt. Die Reaktionslösung wird mit Toluol verdünnt, am Rotationsverdampfer eingeengt und der Rückstand für 5 Minuten am Hochvakuum getrocknet. Das Rohprodukt versetzt man mit 3 ml Trifluoressigsäure und rührt 15 Minuten bei Raumtemperatur. Die Lösung wird mit Toluol verdünnt und anschliessend am Rotationsverdampfer eingeengt (zweimal wiederholt). Der Rückstand wird durch

Verreiben mit Aether in ein Pulver übergeführt und dieses in Wasser gelöst und der pH der Lösung auf 7 eingesetzt. Durch präparative Schichtchromatographie auf DC-Trägerplatten "UP $C_{12}$" (Firma Antec) mit dem Laufmittelsystem Acetonitril/Wasser 1 : 9 wird die 7β-[-(2-Amino-4-thiazolyl)-2-methoxyiminoacetamido]-3-[1-(2-dimethylaminoäthyl)-tetrazol-5-ylthio)-3-cephem-4-carbonsäure rein isoliert (Lyophilisat). Rf-Wert 0,18 ("UP $C_{12}$", Acetonitril/ $H_2O$ 1:9).

Beispiel 11:

Eine Lösung von 19,7 gr (25,8 mMol) 7β-{2-[2-tert.Butoxycarbonylamino)-4-thiazolyl]-2-methoxyiminoacetamido}-3-(1-methyltetrazol-5-ylthio)-3-cephem-4-carbonsäure-diphenylmethylester in 200 ml Chloroform wird auf 0° C gekühlt, unter guter Kühlung mit 5,12 gr (29,7 mMol) m-Chlorperbenzoesäure (90 %-ig) versetzt und 35 Minuten bei 0° C gerührt. Hierauf wird überschüssige Persäure durch Zugabe von Natriumthiosulfat zerstört. Die Reaktionslösung wird mit wässriger Natriumbicarbonatlösung und gesättigter wässriger Kochsalzlösung extrahiert, über Natriumsulfat getrocknet und eingeengt. Der Rückstand wird durch Säulenchromatographie (570 gr. Kieselgel, Toluol/Aethylacetat 1:1) gereinigt und ergibt das 7β-{-2-[2-(tert. Butoxycarbonylamino)-4-thiazolyl]-2-methoxyiminoacetamido}-3-(1-methyltetrazol-5-ylthio)-3-cephem-4-carbonsäure-diphenylmethylester-1β-oxid, DC(Silicagel):Rf = 0.14 (Toluol/Aethylacetat 1:1); IR-Spektrum (Methylenchlorid): charakteristische Banden bei 2,97; 3,12; 5,51; 5,8; 5,94; 6,45μ.

Beispiel 12:

Eine Lösung von 12.2 gr (15,6 mMol) 7β-[2-(2-tert. Butoxycarbonylamino-4-thiazolyl)-2-methoxyiminoacetamido]-3-(1-methyltetrazol-5-ylthio)-3-cephem-4-carbonsäure-diphenylmethylester-1β-oxid in 160 ml Methylenchlorid wird bei 0° C mit 22 ml Trifluoressigsäure versetzt und während 35 Minuten gerührt, dann mit Toluol verdünnt und anschliessend am Rotationsverdampfer eingeengt (zweimal wiederholt). Der Rückstand wird in 47 ml Methylenchlorid gelöst und bei Raumtemperatur mit 5,6 ml (16,8 mMol) Natrium-hexanoat (3-M in Methanol) versetzt und während 45 Minuten gerührt. Die Reaktionslösung wird eingeengt, der Rückstand mit Aether verrieben und das unlösliche Pulver durch Filtration isoliert. Man erhält das 7β-[2-(2-tert.Butoxycarbonylamino-4-thiazolyl)-2-methoxyimino-acetamido]-3-(1-methyltetrazol-5-ylthio)-3-cephem-4-carbonsäure-natriumsalz-1-β-oxid vom Rf-Wert 0.27 (Antecplatten $UPC_{15}$, Acetonitril/Wasser 1 : 3).

Beispiel 13:

Lösung I: Eine Lösung von 10,35 g (69 mMol) Natriumjodid in 30 ml Aceton wird bei Raumtemperatur unter Stickstoff mit 2.56 ml Pivaloyloxymethylchlorid versetzt und 3 Stunden bei dieser Temperatur gerührt. Anschliessend wird unter Stickstoff vom auskristallisierten Natriumchlorid abfiltriert und die klare Lösung weiterverwendet.

Lösung II: Eine Lösung von 3,65 gr (5,75 mMol) 7β-[2-(2-tert. Butoxycarbonylamino-4-thiazolyl)-2-methoxyimino-acetamido]-3-(1-methyltetrazol-5-ylthio)-3-cephem-4-carbon-säure -natriumsalz-1β-oxid und 304 mg (1 mMol) 18-Crown-6 in 18 ml Dimethylformamid wird bei Raumtemperatur unter Stickstoff mit 21,4 ml Lösung I versetzt, bei dieser Temperatur während 2 Std. gerührt, dann mit Aethylacetat

verdünnt, nacheinander 2x mit Wasser und wässriger Natriumchloridlösung gewaschen, über Natriumsulfat getrocknet und im Vakuum eingedampft. Der Rückstand wird durch Säulenchromatographie (130 gr Kieselgel; Toluol/Aethylacetat 1:2) gereinigt. Man erhält das 7ß-[2-(2-tert.Butoxycarbonylamino-4-thiazolyl)-2-methoxyiminoacetamido]-3-(1-methyltetrazol-5-ylthio)-3-cephem-4-carbonsäure-pivaloyloxymethylester-1ß-oxid vom Rf. Wert 0.28 (Silicagel; Toluol/Aethylacetat 1:2).

Beispiel 14:

Eine Lösung von 600 mg (0.82 mMol) 7ß-[2-(2-tert.Butoxycarbonylamino-4-thiazolyl)-2-methoxyiminoacetamido]-3-(1-methyltetrazol-5-ylthio)-3-cephem-4-carbonsäure-pivaloyloxymethylester-1ß-oxid in 6 ml Methylenchlorid und 0.6 ml Dimethylformamid wird auf 0° C gekühlt, mit 0.33 ml (3.6 mMol) Phosphortrichlorid (destilliert) versetzt und bei 0° C 20 Minuten gerührt, dann mit Methylenchlorid verdünnt, nacheinander mit Wasser, wässriger Bicarbonatlösung und wässriger Natriumchloridlösung gewaschen, über Natriumsulfat getrocknet und im Vakuum eingedampft. Der Rückstand wird durch präparative Dickschichtchromatographie an Silicagel mit Toluol/Aethylacetat 1:1 gereinigt. Man erhält den 7ß-[2-(2-terts.Butoxycarbonylamino-4-thiazolyl)-2-methoxyiminoacetamido]-3-(1-methyl-tetrazol-5-ylthio)-3-cephem-4-carbonsäurepivaloyloxymethylester vom Rf-Wert 0.35 (Silicagel; Toluol/Aethylacetat 1:1) als Schaum.
IR-Spektrum (Methylenchlorid): charakteristische Banden bei 2,94; 3,0; 5,57; 5,69; 5,81; 5,93; 6.47 µ.

Beispiel 15:

Eine Mischung von 1,1 g (1,5 mMol) 7β-[2-(2-tert.Butoxycarbonylamino-4-thiazolyl)-2-methoxyiminoacetamido]-3-(1-methyl-tetrazol-5-ylthio)-3-cephem-4-carbonsäurepivaloyloxymethylester und 2.5 ml Anisol wird bei Raumtemperatur mit 10 ml Trifluoressigsäure versetzt und während 15 Minuten gerührt, dann mit Toluol verdünnt und anschliessend am Rotationsverdampfer eingeengt. Der Rückstand wird in Aethylacetat aufgenommen, mit wässriger Bicarbonatlösung gewaschen, über Natriumsulfat getrocknet und am Vakuum eingeengt. Der Rückstand wird mit Aether verrieben und das unlösliche Pulver durch Filtration isoliert. Man erhält den 7β-[2-(2-Amino-4-thiazolyl)-2-methoxyiminoacetamido]-3-(1-methyltetrazol-5-ylthio)-3-cephem-4-carbonsäurepivaloyloxymethylester vom Rf-Wert 0,4 (Silicagel; Aethylacetat); IR-Spektrum (Methylenchlorid): charakteristische Banden bei 2.85; 2.70; 5.58; 5,68; 5,95; 6.21μ.

Beispiel 16:

Eine Lösung von 20,56 g (68,25 mMol) 2-(2-tert.Butoxycarbonylamino-4-thiazolyl)-2-methoxyiminoessigsäure in 210 ml Methylenchlorid und 6,44 ml (79,95 mMol) Pyridin wird bei -15° unter Stickstoff mit 12,6 ml (83,85 mMol) Diäthylphosphobromidat versetzt und 30 Minuten bei dieser Temperatur gerührt. Nach Zugabe von 29,93 g (65,0 mMol) 7β-Amino-3-methansulfonyloxy-3-cephem-4-carbonsäurediphenylmethylester bei -15° wird die Reaktionslösung bei Raumtemperatur während 2 Stunden gerührt, dann mit Chloroform verdünnt, nacheinander mit verdünnter Schwefelsäure, Wasser, wässriger Natriumbicarbonatlösung und wässriger Natriumchloridlösung gewaschen, über Natriumsulfat getrocknet und im Vakuum eingedampft. Der Rückstand wird an 300 g Silicagel mit dem Laufmittelsystem Toluol/Aethylacetat 1:1 chromatogra

phiert. Man erhält den 7β-[2-(2-tert.Butoxycarbonylamino-4-thiazolyl)-2-methoxyiminoacetamido]-3-methansulfonyloxy-3-cephem-4-carbonsäurediphenylmethylester vom Rf-Wert 0,42 (Silicagel;Toluol/Aethylacetat 1:1) als Schaum. IR-Spektrum (Methylenchlorid) charakteristische Banden bei 2,94; 3,06; 5,56; 5,77; 5,91; 6,44μ. Als Nebenprodukt wird wenig von 7β-[2-(2-tert.Butoxycarbonylamino-4-thiazolyl)-2-methoxyiminoacetamido]-3-methansulfonyloxy-2-cephem-4-carbonsäurediphenylmethylester vom Rf-Wert 0,30 (Silicagel; Toluol/Aethylacetat 1:1) isoliert. IR-Spektrum (Methylenchlorid): charakteristische Banden bei 2,94; 3,06; 5,56; 5,78; 5,90; 6,43μ.

Beispiel 17:

Eine Suspension von 531 mg (7,8 mMol) Natriumäthylat in 36 ml Tetrahydrofuran wird bei Raumtemperatur unter Stickstoff mit 1,27 g (9,6 mMol) 5-Mercapto-2-methyl-1,3,4-thiadiazol versetzt und bei gleicher Temperatur während 20 Minuten gerührt. Zur klaren Lösung werden anschliessend 4,46 g (6,0 mMol) 7β-[2-(2-tert.Butoxycarbonylamino-4-thiazolyl)-2-methoxyiminoacetamido]-3-methansulfonyloxy-3-cephem-4-carbonsäure-diphenylmethylester gegeben und bei Raumtemperatur während 90 Minuten gerührt. Das Lösungsmittel wird im Vakuum abgedampft, der Rückstand in Aethylacetat aufgenommen und die Lösung mit wässriger Natriumbicarbonatlösung und wässriger Natriumchloridlösung gewaschen, über Natriumsulfat getrocknet und am Vakuum eingedampft. Man erhält ein Gemisch von 7β-[2-(2-tert.Butoxycarbonylamino-4-thiazolyl)-2-methoxyiminoacetamido]-3-(2-methyl-1,3,4,thiadiazol-5-ylthio)-3-cephem-4-carbonsäurediphenylmethylester vom Rf-Wert 0,20 und 7β-[2-(2-tert.Butoxycarbonylamino-4-thiazolyl)-2-methoxyiminoacetamido]-3-(2-methyl-1,3,4-thiadiazol-5-ylthio)-2-cephem-4-carbonsäurediphenylmethylester vom Rf-Wert 0,14 (Silicagel: Toluol/Aethylacetat 1:1) als Schaum. IR-Spektrum (Methylenchlorid):

charakteristische Banden bei 2,94; 3,07; 5,60; 5,73; 5,79; 5,93; 6,45μ.

Beispiel 18:

Eine Lösung von 4,64g (5,95 mMol) 7β-[2-(2-tert.Butoxycarbonylamino-4-thiazolyl)-2-methoxyiminoacetamide]-3-(2-methyl-1,3,4-thiadiazol-5-ylthio)-2-cephem-4-carbonsäurediphenylmethylester in 47 ml Chloroform wird auf 0° gekühlt, unter guter Kühlung mit 1,21 g (6,31 mMol) m-Chlorperbenzoesäure (90 %ig) versetzt und 1/4 Stunde gerührt. Hierauf wird überschüssige Persäure durch Zugabe von Natriumthiosulfat zerstört. Die Reaktionslösung wird mit wässriger Natriumbicarbonat-und gesättigter wässriger Natriumchloridlösung extrahiert, über Natriumsulfat getrocknet und eingeengt. Der Rückstand wird an 150 g Silicagel mit dem Laufmittel Aethylacetat chromatographiert. Man erhält das 7β-[2-(2-tert.Butoxycarbonylamino-4-thiazolyl)-2-methoxyiminoacetamido]-3-(2-methyl-1,3,4-thiadiazol-5-ylthio)-3-cephem-4-carbonsäurediphenylmethylester-1β-oxid vom Rf-Wert 0,21 (Silicagel: Aethylacetat) als Schaum. IR-Spektrum (Methylenchlorid) charakteristische Banden bei 2,95; 3,14; 5,52; 5,77; 5,89; 6,44μ.

Beispiel 19:

Eine Lösung von 2,35 g (2,95 mMol) 7β-[2-(2-tert. Butoxycarbonylamino-4-thiazolyl)-2-methoxyiminoacetamido]-3-(2-methyl-1,3,4-thiadiazol-5-ylthio)-3-cephem-4-carbonsäurediphenylmethylester-1β-oxid in 35 ml Methylenchlorid und 2,4 ml Dimethylformamid wird auf 0° gekühlt, mit 1,03 ml (11,81 mMol)Phosphortrichlorid (destilliert) versetzt und 30 Minuten gerührt. Die Reaktionslösung wird mit wässriger Natriumbicarbonatlösung und gesättigter Natriumchloridlösung extrahiert, die organische Phase über Natriumsulfat getrocknet und eingedampft. Man erhält den 7β-[2-(2-tert.

Butoxycarbonylamino-4-thiazolyl)-2-methoxyiminoacetamido]-3-(2-methyl-1,3,4-thiadiazol-5-ylthio)-3-cephem-4-carbonsäure-diphenylmethylester vom Rf-Wert 0,20 (Silicagel: Toluol/Aethylacetat 1:1) als Schaum. IR-Spektrum (Methylenchlorid) charakteristische Banden bei 2,94; 3,07; 5,60; 5,78; 5,93; 6,44 μ.

Beispiel 20:

Eine Lösung von 1,19 g (1,53 mMol) 7β-[2-(2-tert. Butoxycarbonylamino-4-thiazolyl)-2-methoxyiminoacetamido]-3-(2-methyl-1,3,4-thiadiazol-5-ylthio)-3-cephem-4-carbonsäurediphenylmethylester in 2,4 ml Methylenchlorid und 0,85 ml Anisol wird bei Raumtemperatur mit 11,5 ml Trifluoressigsäure versetzt und während 25 Minuten gerührt. Die Reaktionslösung wird anschliessend in 500 ml Diäthyläther/Hexan 1:2 getropft und das unlösliche Pulver durch Filtration isoliert. Dieses wird in wässrigem Methanol gelöst, auf pH 7 gestellt und lyophilisiert. Der Rückstand wird an 30 g silyliertem Silicagel ("UPC12", Firma Antec AG) mit dem Laufmittelsystem Acetonitril/Wasser 1:9 chromatographiert. Man erhält das Natriumsalz der 7β-[2-(2-Amino-4-thiazolyl)-2-methoxyiminoacetamido]-3-(2-methyl-1,3,4-thiadiazol-5-ylthio)-3-cephem-4-carbonsäure vom Rf-Wert 0,29 (silyliertes Silicagel, "UPC12": Acetonitril/Wasser 1:6) als beiges Pulver.

Beispiel 21:

Eine Suspension von 885 mg (13,0 mMol) Natriumäthylat in 60 ml Tetrahydrofuran wird bei Raumtemperatur unter Stickstoff mit 1,89 g (16,0 mMol, 2-Mercapto-1,3,4-thiadiazol versetzt und bei gleicher Temperatur während 20 Minuten gerührt. Zur klaren Lösung werden anschliessend 7,44 g (10,0 mMol) 7β-[2-(2-tert.Butoxycarbonylamino-4-thiazolyl)-2-methoxyiminoacetamido]-3-methansulfonyloxy-3-cephem-4-carbonsäurediphenylmethylester gegeben und bei Raumtemperatur während 45 Minuten gerührt. Das Lösungsmittel wird im Vakuum abgedampft, der Rückstand in Aethylacetat aufgenommen und die Lösung mit wässriger Natriumbicarbonatlösung und wässriger Natriumchloridlösung gewaschen, über Natriumsulfat getrocknet und am Vakuum eingedampft. Man erhält ein Gemisch von 7β-[2-(2-tert.Butoxycarbonylamino-4-thiazolyl)-2-methoxyiminoacetamido-3-(1,3,4-thiadiazol-5-ylthio)-3-cephem-4-carbonsäurediphenylmethylester vom Rf-Wert 0,25 und 7β-[2-(2-tert.Butoxycarbonylamino-4-thiazolyl)-2-methoxyiminoacetamido]-3-(1,3,4-thiadiazol-5-ylthio)-2-cephem-4-carbonsäurediphenylmethylester vom Rf-Wert 0,16 (Silicagel: Toluol/Aethylacetat 1:1) als Schaum. IR-Spektrum (Methylenchlorid): charakteristische Banden bei 2,94; 3,07; 5,61; 5,74; 5,78; 5,93; 6,44μ.

Beispiel 22:

Eine Lösung von 2,28 g (2,98 mMol) 7β-[2-(2-tert. Butoxycarbonylamino-4-thiazolyl)-2-methoxyiminoacetamido]-3-(1,3,4-thiadiazol-5-ylthio)-2-cephem-4-carbonsäurediphenylmethylester in 20 ml Chloroform wird auf 0° gekühlt, unter guter Kühlung mit 606 mg (3,16 mMol) m-Chlorperbenzoesäure (90 %ig) versetzt und 1/4 Stunde gerührt. Hierauf wird überschüssige Persäure durch Zugabe von Natriumthiosulfat zerstört. Die Reaktionslösung wird mit wässriger Natriumbicarbonat- und gesättigter, wässriger Natriumchloridlösung extrahiert, über Natriumsulfat getrocknet und eingeengt. Der Rückstand wird an 150 g Silicagel mit dem Laufmittel Aethylacetat chromatographiert. Man erhält das 7β-[2-(2-tert. Butoxycarbonylamino-4-thiazolyl)-2-methoxyiminoacetamido]-3-(1,3,4-thiadiazol-5-ylthio)-3-cephem-4-carbonsäurediphenylmethylester-1β-oxid vom Rf-Wert 0,23 (Silicagel: Aethylacetat) als Schaum. IR-Spektrum (Methylenchlorid):

charakteristische Banden bei 2,94; 3,13; 5,51; 5,77; 5,90; 6,43 μ.

Beispiel 23:

Eine Lösung von 2,50 g (3,20 mMol) 7β-[2-(2-tert.Butoxycarbonylamino-4-thiazolyl)-2-methoxyiminoacetamido]-3-(1,3,4-thiadiazol-5-ylthio)-3-cephem-4-carbonsäurediphenylmethylester-1β-oxid in 37,5 ml Methylenchlorid und 2,5 ml Dimethylformamid wird auf 0° gekühlt, mit 1,12 ml (12,8 mMol) Phosphortichlorid (destilliert) versetzt und 30 Minuten gerührt. Die Reaktionslösung wird mit wässriger Natriumbicarbonatlösung und gesättigter, wässriger Natriumchloridlösung extrahiert, die organische Phase über Natriumsulfat getrocknet und eingedampft. Man erhält den 7β-[2-(2-tert.Butoxycarbonylamino-4-thiazolyl)-2-methoxyiminoacetamido]-3-(1,3,4-thiadiazol-5-ylthio)-3-cephem-4-carbonsäurediphenylmethylester vom Rf-Wert 0,25 (Silicagel: Toluol/Aethylacetat 1:1) als Schaum. IR-Spektrum (Methylenchlorid):charakteristische Banden bei 2,94, 3,07; 5,60; 5,78; 5,93; 6,44 μ.

Beispiel 24:

Eine Lösung von 1,65 g (2,15 mMol) 7β-[2-(2-tert.Butoxycarbonylamino-4-thiazolyl)-2-methoxyiminoacetamido]-3-(1,3,4-thiadiazol-5-ylthio)-3-cephem-4-carbonsäurediphenylmethylester in 3,36 ml Methylenchlorid und 1,2 ml Anisol wird bei Raumtemperatur mit 16 ml Trifluoressigsäure versetzt und während 25 Minuten gerührt. Die Reaktionslösung wird anschliessend in 660 ml Diaethyläther/Hexan 1:2 getropft und das unlösliche Pulver durch Filtration isoliert. Dieses wird in wässrigem Methanol gelöst, auf pH 7 gestellt und lyophilisiert. Der Rückstand wird an 30 g silyliertem Silicagel ("UPC12", Firma Antec AG) mit dem Laufmittelsystem Acetonitril/Wasser 1 : 9 chromatographiert. Man erhält das Natriumsalz der 7β-[2-(2-Amino-4-thiazolyl)-2-methoxyiminoacetamido]-3-(1,3,4-thiadiazol-5-ylthio)-3-cephem-4-carbonsäure vom Rf-Wert 0,38 (silyliertes Silicagel, "UPC12": Acetonitril/Wasser 1:6) als beiges Pulver.

Beispiel 25:

Eine Suspension von 266 mg (3,91 mMol) Natriumäthylat in 17 ml Tetrahydrofuran wird bei Raumtemperatur unter Stickstoff mit 1,57 g (4,81 mMol) 1-Diphenylmethyloxycarbonylmethyl-5-mercaptotetrazol versetzt und bei gleicher Temperatur während 20 Minuten gerührt. Zur klaren Lösung werden anschliessend 2,23 g (3,00 mMol) 7β-[2-(2-tert. Butoxycarbonylamino-4-thiazolyl)-2-methoxyiminoacetamido]-3-methansulfonyloxy-3-cephem-4-carbonsäurediphenylmethylester gegeben und bei 40° während 21 Stunden gerührt. Das Lösungsmittel wird im Vakuum abgedampft, der Rückstand in Aethylacetat aufgenommen und die Lösung mit wässriger Natriumbicarbonatlösung und wässriger Natriumchloridlösung gewaschen, über Natriumsulfat getrocknet und am Vakuum eingedampft. Der Rückstand wird an 200 g Silicagel mit dem Laufmittelsystem Toluol/Aethylacetat 1:1 chromatographiert. Man erhält den 7β-[2-(2-tert.Butoxycarbonylamino-4-thiazolyl)-2-methoxyiminoacetamido]-3-(1-diphenylmethoxycarbonylmethyltetrazol-5-ylthio)-3-cephem-4-carbonsäurediphenyl-methylester vom Rf-Wert 0,54 (Silicagel: Toluol/Aethylacetat 1:1) als Schaum. IR-Spektrum (Methylenchlorid): charakteristische Banden bei 2,94; 3,05; 5,56; 5,68; 5,78; 5,90; 6,44 μ.

Beispiel 26:

Eine Lösung von 1,23 g (1,23 g (1,26 in Mol) 7β-[2-(2-tert. Butoxycarbonylamino-4-thiazolyl)-2-methoxyiminoacetamido]-3-(1-diphenylmethyloxycarbonylmethyl-tetrazol-5-ylthio)-3-cephem-4-Carbonsäurediphenylmethylester in 1,9 ml Methylenchlorid und 0,7 ml Anisol wird bei Raumtemperatur mit 9,3 ml Trifluoressigsäure versetzt und während 25 Minuten gerührt. Die Reaktionslösung wird anschliessend in 400 ml Diaethylaether/Hexan 1 : 2 getropft und das unlösliche Pulver durch Filtration isoliert. Dieses wird in wässrigem Methanol gelöst, auf pH 7 gestellt und lyophilisiert. Der Rückstand wird an 30 g silyliertem Silicagel ("UPC12, Firma Antec AG") mit dem Laufmittel Wasser chromatographiert. Man erhält das Dinatriumsalz der 7β-[2-(2-Amino-4-thiazolyl)-2-methoxyiminoacetamido]-3-(1-carboxymethyletrazol-5-ylthio)-3-cephem-4-carbonsäure vom Rf-Wert 0, 33 (silyiertes Silicagel, "UPC12": Acetonitril/Wasser 1:30) als beiges Pulver.

Beispiel 27:

a) Eine Suspension von 1,75 g (15,6 mMol) Kalium-tert.butylat in 68 ml Tetrahydrofuran wird bei Raumtemperatur unter Stickstoff mit 2,29 g (19,21 mMol) 2-Mercaptothiazolin versetzt und bei gleicher Temperatur während 20 Minuten gerührt. Zur Lösung werden anschliessend 8,93 g (12,0 mMol) 7β-[2-(2-tert.Butoxycarbonylamino-4-thiazolyl)-2-methoxyiminoacetamido]-3-methansulfonyloxy-3-cephem-4-carbonsäurediphenylmethylester gegeben und bei Raumtemperatur während 21 Stunden gerührt. Das Lösungsmittel wird am Vakuum abgedampft, der Rückstand in Aethylacetat aufgenommen und die Lösung mit wässriger Natriumbicarbonatlösung und wässriger Natriumchloridlösung gewaschen, über Natriumsulfat getrocknet und am Vakuum eingedampft. Der Rückstand wird an 700 g Silicagel mit dem Laufmittelsystem Toluol/Methylacetat 2:1 chromatographiert. Man erhält den 7β-[2-(2-tert.Butoxycarbonylamino-4-thiazolyl)-2-methoxyiminoacetamido]-3-(thiazolin-2-ylthio)-2-cephem-4-carbonsäure-diphenylmethylester vom Rf-Wert 0,25 (Silicagel-Toluol/Aethylacetat 1:1) als Schaum. IR-Spektrum (Methylthiochlorid): charakteristische Banden bis 2,94; 3,07; 5,60; 5,73; 5,79; 5,93; 6,45µ.

Das Ausgangsmaterial kann wie folgt hergestellt werden:

b) Eine Lösung von 16,65 g (0,104 Mol) 1-Carboxymethyl-5-mercaptotetrazol (W.J. Gottstein, M.A. Kaplan, J.A. Cooper, V.H. Silver, S.J. Nachfolger, A.P. Granatek, Journal of Antibiotics 29 (II), 1226 (1976)) in 200 ml Dioxan wird bei Raumtemperatur mit 24,24 g (0,125 Mol) Diphenyldiazomethan versetzt und bei gleicher Temperatur noch 60 Minuten gerührt. Das Lösungsmittel wird im Vakuum abgedampft. Der Rückstand wird durch präparative Dickschichtchromatographie an Silicagel mit Toluol/Aethylacetat/Essigsäure: 73 : 25 : 2 gereinigt. Man erhält das 1-Diphenylmethoxycarbonylmethyl-5-mercaptotetrazol vom Rf-Wert 0,44 (Silicagel: Aethylacetat Essigsäure 98:2) als amorphes Pulver. IR-Spektrum (Methylenchlorid): charakteristische Banden bis 2,96; 3.30; 3,42; 5,72; 6,25; 6,70; 6,74µ.

Beispiel 28:

Eine Lösung von 5,48 g (7,15 mMol) 7β-[2-(2-tert.Butoxycarbonylamino-4-thiazolyl)-2-methoxyiminoacetamido]-3-(thiazolin-2-ylthio)-2-cephem-4-carbonsäurediphenylmethylester in 55 ml Chloroform wird auf 0° gekühlt, unter guter Kühlung mit 2,91 g (15,2 mMol) m-Chlorperbenzoesäure (90 %ig) versetzt und 1/4 Stunde gerührt. Hierauf wird überschüssige Persäure durch Zugabe von Natriumthiosulfat zerstört. Die Reaktionslösung wird mit wässrigem Natriumchloridlösung extrahiert, über Natriumsulfat getrocknet und eingeengt. Der Rückstand wird an 150 g Silicagel mit dem Laufmittelsystem Aceton/Aethylacetat 1:1 chromatographiert. Man erhält das 7β-[2-(2-tert.Butoxycarbonylamino-4-thiazolyl)-2-methoxyiminoacetamido]-3-(1-oxido-thiazolin-2-ylthio)-3-cephem-4-carbonsäurediphenylmethylester-1β-oxid vom Rf-Wert 0,25 (Silicagel: Aceton/Aethylacetat 1:1) als Schaum. IR-Spektrum (Methylenchlorid): charakteristische Banden bei 2,95; 3,13; 5,52; 5,78; 5,91; 6,44μ.

Beispiel 29:

Eine Lösung von 4,36 g (5,45 mMol) 7β-[2-(2-tert.Butoxycarbonylamino-4-thiazolyl)-2-methoxyiminoacetamido]-3-(1-oxido-thiazolin-2-ylthio)-3-cephem-4-carbonsäurediphenylmethylester-1β-oxid in 65 ml Methylenchlorid und 4,3 ml Dimethylformamid wird auf 0°gekühlt, mit 3,8 ml (43,6 mMol) Phosphortrichlorid (destilliert) versetzt und 30 Minuten gerührt. Die Reaktionslösung wird mit wässriger Natriumbicarbonatlösung und gesättigter Natriumchloridlösung extrahiert, die organische Phase über Natriumsulfat getrocknet und eingedampft. Man erhält den 7β-[2-(2-tert. Butoxycarbonylamino-4-thiazolyl)-2-methoxyiminoacetamido]-3-(thiazolin-2-ylthio)-3-cephem-4-carbonsäurediphenylmethylester vom Rf-Wert 0,30 (Silicagel: Toluol/Aethylacetat 1:1) als Schaum. IR-Spektrum (Methylenchlorid):charakteristische Banden bei 2,94; 3,06; 5,57; 5,77; 5,94; 6,44μ.

Beispiel 30:

Eine Lösung von 4,30 g (5,61 mMol) 7β-[2-(2-tert.Butoxycarbonylamino-4-thiazolyl)-2-methoxyiminoacetamido]-3-(thiazolin-2-ylthio)-3-cephem-4-carbonsäurediphenylmethylester in 8,6 ml Methylenchlorid und 3,1 ml Anisol wird bei Raumtemperatur mit 39 ml Trifluoressigsäure versetzt und

während 25 Minuten gerührt. Die Reaktionslösung wird anschliessend in 1,5 l Diaethyläther/Hexan 1:2 getropft und das unlösliche Pulver durch Filtration isoliert. Dieses wird in wässrigem Methanol gelöst, auf pH 7 gestellt und lyophilisiert. Der Rückstand wird an 30 g silyliertem Silicagel ("UPC12", Firma Antec AG) mit dem Laufmittel Wasser chromatographiert. Man erhält das Natriumsalz der 7β-[2-(2-Amino-4-thiazolyl)-2-methoxyiminoacetamido]-3-(thiazolin-2-ylthio)-3-cephem-4-carbonsäure vom Rf-Wert 0,25 (silyliertes Silicagel, "UPC12": Acetonitril/Wasser 1 : 6) als beiges Pulver.

Beispiel 31: Analog den vorstehenden Beispielen können ausgehend von entsprechenden Ausgangsmaterialien die folgenden Verbindungen erhalten werden:

7β-[2-(2-Amino-4-thiazolyl)-2-methoxyiminoacetamido]-3-[1-(2-carboxyäthyl)-tetrazol-5-ylthio]-3-cephem-4-carbonsäure;

7β-[2-(2-Amino-4-thiazolyl)-2-methoxyiminoacetamido]-3-(1-sulfomethyltetrazol-5-ylthio)-3-cephem-4-carbonsäure;

7β-[2-(2-Amino-4-thiazolyl)-2-methoxyiminoacetamido]-3-(1,2,3-triazol-4-ylthio)-3-cephem-4-carbonsäure;

7β-[2-(2-Amino-4-thiazolyl)-2-methoxyiminoacetamido]-3-(sulfomethyl-1,3,4-oxadiazol-5-ylthio)-3-cephem-4-carbonsäure;

7β-[2-(2-Amino-4-thiazolyl)-2-methoxyiminoacetamido]-3-(3-methyl-3-oxido-6-pyridazinylthio)-3-cephem-4-carbonsäure;

7β-[2-(2-Amino-4-thiazolyl)-2-methoxyiminoacetamido]-3-(3-hydroxy-6-pyridazinylthio)-3-cephem-4-carbonsäure;

7β-[2-(2-Amino-4-thiazolyl)-2-methoxyiminoacetamido]-3-tetrazolo[4,5-b]pyridazin-6-ylthio-3-cephem-4-carbonsäure, sowie ihre Natriumsalze.

Beispiel 32: Kapseln, enthaltend 0,250 g 7β-[2-(2-Amino-4-thiazolyl)-2-methoxyiminoacetamido]-3-(1-methyltetrazol-5-ylthio)-3-cephem-4-carbonsäure-natriumsalz werden wie folgt hergestellt:

Zusammensetzung (für 100'000 Kapseln):

| | |
|---|---|
| 7β-[2-(2-Amino-4-thiazolyl)-2-methoxyiminoacetamido]-3-(1-methyltetrazol-5-ylthio)-3-cephem-4-carbonsäure-natriumsalz | 25'000 g |
| Weizenstärke | 2'500 g |
| Magnesiumstärke | 1'000 g |

Das 7β-[2-(2-Amino-4-thiazolyl)-2-methoxyiminoacetamido]-3-(1-methyltetrazol-5-ylthio)-3-cephem-4-carbonsäure-natriumsalz, die Weizenstärke und das Magnesiumstearat werden gut miteinander vermischt und in Kapseln Nr. 1 abgefüllt.

Beispiel 33: Kapseln enthaltend 0,5 g 7β-[2-(2-Amino-4-thiazolyl)-2-methoxyiminoacetamido]-3-(1-methyltetrazol-5-ylthio)-3-cephem-4-carbonsäurenatriumsalz werden wie folgt hergestellt:

Zusammensetzung (für 2000 Kapseln):

| | |
|---|---|
| 7β-[2-(2-Amino-4-thiazolyl)-2-methoxyiminoacetamido]-3-(1-methyltetrazol-5-ylthio)-3-cephem-4-carbonsäurenatriumsalz | 1000,00 g |
| Polyvinylpyrrolidon | 15,00 g |
| Maisstärke | 115,00 g |
| Magnesiumstearat | 20,00 g |

Man befeuchtet das 7β-[2-(2-Amino-4-thiazolyl)-2-methoxyiminoacetamido]-3-(1-methyltetrazol-5-ylthio)-3-cephem-4-carbonsäure-natriumsalz mit 300 ml einer Lösung des Polyvinylpyrrolidons in 95%-igem Aethanol, treibt das Gemisch durch ein Sieb mit 3 mm Maschenweite und trocknet das Granulat unter vermindertem Druck bei 40-50°. Man siebt durch ein Sieb mit 0,8 mm Maschenweite, gibt die Maisstärke und das Magnesiumstearat zu, vermischt und füllt das Gemisch in Steckkapseln (Grösse 0) ab.

Beispiel 34: Trockenampullen oder Vials, enthaltend 0,5 g 7β-[2-(2-Amino-4-thiazolyl)-2-methoxyiminoacetamido]-3-(1-methyltetrazol-5-ylthio)-3-cephem-4-carbonsäure-natriumsalz werden wie folgt hergestellt:

Zusammensetzung: (für 1 Ampulle oder Vial)

| | |
|---|---|
| 7β-[2-(2-Amino-4-thiazolyl)-2-methoxyiminoacetamido]-3-(1-methyltetrazol-5-ylthio)-3-cephem-4-carbonsäure-natriumsalz | 0,5 g |
| Mannit | 0,05 g |

Eine sterile wässrige Lösung des 7β-[2-(2-Amino-4-thiazolyl)-2-methoxyiminoacetamido]-3-(1-methyltetrazol-5-ylthio)-3-cephem-4-carbonsäure-natriumsalzes und des Mannits wird unter aseptischen Bedingungen in 5 ml.-Ampullen oder 5 ml.-Vials verschlossen und geprüft.

Patentansprüche

1. 7β-Aminothiazolyl-acetamido-3-cephem-4-carbonsäureverbindungen der Formel

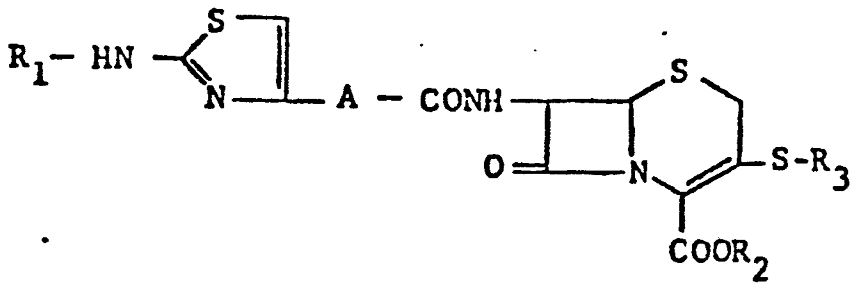

(I)

worin $R_1$ Wasserstoff, Niederalkyl oder eine Aminoschutzgruppe, A Methylen oder durch gegebenenfalls geschütztes Amino, Hydroxy, gegebenenfalls geschütztes Sulfo, Oxo, gegebenenfalls geschütztes Hydroxyimino oder Methoxyimino substituiertes Methylen, $R_2$ Wasserstoff oder einen die Carboxylgruppe veresternden Rest und $R_3$ einen Heterocyclylrest darstellen, und Salze von solchen Verbindungen mit salzbildenden Gruppen.

2. Verbindungen der Formel (I) nach Patentanspruch 1, worin $R_1$ Wasserstoff, Niederalkyl, Niederalkanoyl oder 2-Halogenniederalkanoyl bedeutet, A Methylen, Aminomethylen, Hydroxymethylen, Sulfomethylen, Hydroxyiminomethylen oder Methoxyiminomethylen darstellt, $R_2$ Wasserstoff und $R_3$ eine gegebenenfalls substituierte Triazolyl, Tetrazolyl-, Thiazolinyl-, Thiadiazolyl-, Oxadiazolyl-, Pyridazinyl- oder Tetrazolopyridazinylgruppe bedeutet, und ihre Salze, sowie die entsprechenden Verbindungen mit geschützten funktionellen Gruppen.

3. Verbindungen der Formel (I) nach Patentanspruch 1, worin $R_1$ Wasserstoff oder 2-Halogenniederalkanoyl bedeutet A Hydroxyiminomethylen oder Methoxyiminomethylen darstellt $R_2$ Wasserstoff und $R_3$ 1-Methyl-1H-tetrazol-5-yl, 1-Carboxymethyl-1H-tetrazol-5-yl, 1-(2-Carboxyäthyl)-1H-tetrazol-5-yl, 1-(2-Dimethylaminoäthyl)-1H-tetrazol-5-yl, 1-Sulfomethyl-1H-tetrazol-5-yl, Thiazolin-2-yl, 1,3,4-Thiadiazol-5-yl, 2-Methyl-1,3,4-thiadiazol-5-yl oder 1,2,3-Triazol-4-yl bedeutet, und ihre Salze, sowie die entsprechenden Verbindungen mit geschützten funktionellen Gruppen.

4. 7β-[2-(2-Amino-4-thiazolyl)-methoxyiminoacetamido]-3-(1-methyltetrazol-5-ylthio)-3-cephem-4-carbonsäure, 7β-[2-(2-Amino-4-thiazolyl)-2-methoxyimonoacetamido]-3-[1-(2-dimethylaminoäthyl)-tetrazol-5-ylthio]-3-cephem-4-carbonsäure, 7β-[2-(2-Amino-4-thiazolyl)-2-methoxyimino-acetamido]-3-(1-methyltetrazol-5-ylthio)-3-cephem-4-carbon säure-pivaloyloxymethylester, 7β-[2-(2-Amino-4-thiazolyl)-2-methoxyiminoacetamido]-3-(2-methyl-1,3,4-thiadiazol-5-ylthio)-3-cephem-4-carbonsäure, 7β-[2-(2-Amino-4-thiazolyl 2-methoxyiminoacetamido]-3-(1,3,4-thiadiazol-5-ylthio)-3-cephem-4-carbonsäure, und 7β-[2-(2-Amino-4-thiazolyl)-2-methoxyiminoacetamido]-3-(2-thiazolinylthio)-3-cephem-4-carbonsäure und ihre pharmazeutisch annehmbaren Salze nach Patentanspruch 1.

5. 7β-[2-(2-Amino-4-thiazolyl)-2-methoxyiminoacet-amido]-3-(1-carboxymethyltetrazol-5-ylthio)-3-cephem-4-carbonsäure und ihre pharmazeutisch annehmbaren Salze nach Patentanspruch 1.

6. Die Natriumsalze der Verbindungen nach einem der Patentansprüche 1 - 6.

7. Pharmazeutische Präparate enthaltend eine der Verbindungen der Patentansprüche 1 - 6.

8. Verwendung einer Verbindung gemäss einem der Patentansprüche 1 - 6 zur Behandlung von bakteriellen Infektionen.

9. Verfahren zur Herstellung von 7β-Aminothiazolylacetamido-3-cephem-4-carbonsäure-verbindungen der Formel

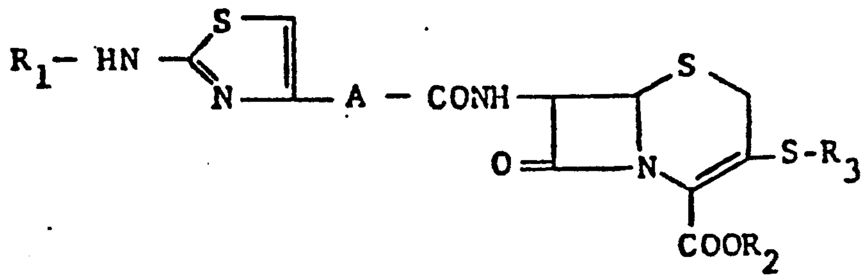

(I)

worin $R_1$ Wasserstoff, Niederalkyl oder eine Aminoschutzgruppe, A Methylen oder durch gegebenenfalls geschütztes Amino, Hydroxy, gegebenenfalls geschütztes Sulfo, Oxo, gegebenenfalls geschütztes Hydroxyimino oder Methoxyimino substituiertes Methylen, $R_2$ Wasserstoff oder einen die Carboxylgruppe veresternden Rest und $R_3$ einen Heterocyclyrest darstellen, und Salzen von solchen Verbindungen mit salzbildenden Gruppen, dadurch gekennzeichnet, dass man

a) die 7β-Aminogruppe in einer Verbindung der Formel

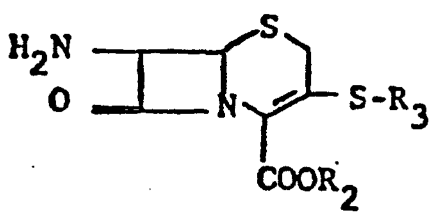

(II),

worin die Aminogruppe gegebenenfalls durch eine die Acylierung erlaubende Gruppe substituiert ist, und worin $R_2$ Wasserstoff oder einen die Carboxylgruppe veresternden Rest

darstellt, durch Behandeln mit einem den Acylrest einer Carbonsäure der Formel

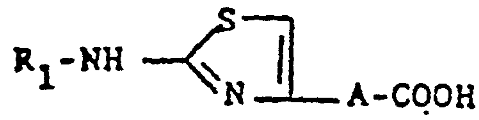

(III)

einführenden Acylierungsmittel, worin gegebenenfalls vorhandene funktionelle Gruppe in geschützter Form vorliegen, acyliert, oder

b) eine Verbindung der Formel

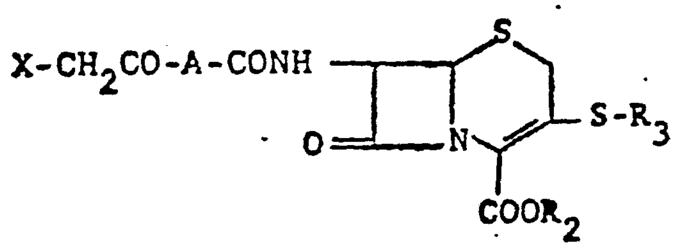

(IV),

worin X Halogen bedeutet, und worin funktionelle Gruppen gegebenenfalls geschützt sind, oder ein Salz davon, mit einem Thioharnstoff der Formel $R_1$-NH-CS-$NH_2$ oder einem Salz davon kondensiert, oder

c) eine Verbindung der Formel

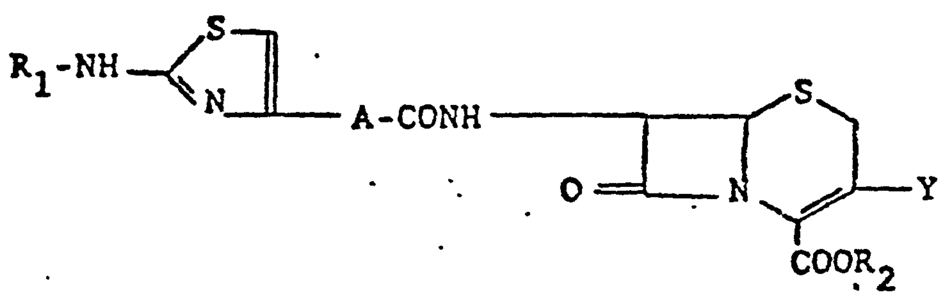

(V)

worin Y eine gegen den Thiolrest -S-$R_3$ austauschbare Gruppe darstellt, und worin funktionelle Gruppen gegebenenfall geschützt sind, in Gegenwart einer Base mit einem Thiol der Formel HS-$R_3$, wobei in $R_3$ vorhandene funktionelle Grup

pen gegebenenfalls geschützt sind, und gegebenenfalls mit einer Säure behandelt, oder

d) eine isomere Verbindung der Formel

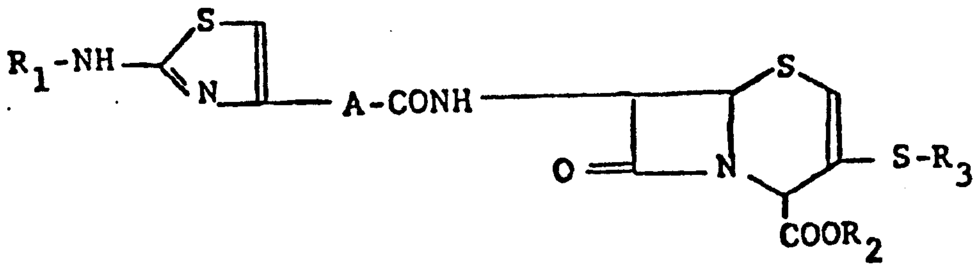

(VI),

isomerisiert, und, wenn erwünscht, in einer erhaltenen Verbindung einen Rest $R_1$, $R_2$, $R_3$ oder A in einen anderen Rest $R_1$, $R_2$, $R_3$ bzw. A überführt, und/oder, wenn erwünscht, eine erhaltene Verbindung in ein Salz oder ein erhaltenes Salz in eine freie Verbindung oder in ein anderes Salz überführt.